# EUROPEAN PATENT SPECIFICATION

(11) **EP 2 167 186 B1**
(45) Date of publication and mention of the grant of the patent: **19.10.2016**
(21) Application number: 07844946.9
(22) Date of filing: 07.11.2007
(51) Int. Cl.: A61N 1/36

(54) **VIDEO PROCESSING UNIT FOR A VISUAL PROSTHETIC APPARATUS**
VIDEOAUFBEREITUNGSGERÄT FÜR EIN SEHPROTHESENGERÄT
UNITÉ DE TRAITEMENT VIDÉO POUR UN APPAREIL DE PROTHÈSE VISUELLE

(30) Priority: 14.06.2007 WO PCT/US2007/013918; 27.07.2007 US 881433; 15.08.2007 US 291134
(43) Date of publication of application: 31.03.2010
(73) Proprietor: Second Sight Medical Products, Inc., Sylmar, CA 91342 (US)
(72) Inventor: MCCLURE, Kelly H., Simi Valley, CA 93063 (US); ROY, Arup, Valencia, CA 91350 (US); CASTRO, Richard Agustin, Santa Monica, CA 90403 (US); YADAV, Sumit, Los Angeles, CA 90024 (US); DAI, Rongqing, Valencia, CA 91354 (US); GREENBERG, Robert J., Los Angeles, CA 90068 (US); CHANG, Da-Yu, Rowland Heights, CA 91748 (US); WU, Xiaofan, Saugus, CA 91350 (US); LOFTIN, Scott, Rosamond, CA 93560 (US); MCCORD, Susan, Santa Monica, CA 90403 (US)
(74) Representative: Carter, Stephen John
(86) International application number: PCT/US2007/083944
(87) International publication number: WO 2008/153592

(56) References cited:
- WO-A-96/01665
- WO-A-2007/064916
- GB-A- 2 019 625
- US-A1- 2004 230 247
- US-A1- 2005 201 585
- US-A1- 2006 003 809
- US-A1- 2006 058 857
- US-B2- 7 278 734
- ANONYMOUS: 'Nokia Bluetooth Headset BH-501 User Guide', [Online] 01 January 2006, XP055265893 Retrieved from the Internet: <URL:http://nds1.webapps.microsoft.com/phon es/files/main_page/Nokia_BH-501_UG_en.pdf> [retrieved on 2016-04-15]
- [Online] 24 November 2006, Retrieved from the Internet: <URL:http://tweakers.net/pricewatch/148073/ nokia-bh-501-bluetooth-headset/specificatie s/> [retrieved on 2016-04-15]

## Description

### FIELD

The present disclosure relates to visual prostheses configured to provide neutral stimulation for the creation of artificial vision.

### BACKGROUND

In 1755 LeRoy passed the discharge of a Leyden jar through the orbit of a man who was blind from cataract and the patient saw "flames passing rapidly downwards." Ever since, there has been a fascination with electrically elicited visual perception. The general concept of electrical stimulation of retinal cells to produce these flashes of light or phosphenes has been known for quite some time. Based on these general principles, some early attempts at devising a prosthesis for aiding the visually impaired have included attaching electrodes to the head or eyelids of patients. While some of these early attempts met with some limited success, these early prosthetic devices were large, bulky and could not produce adequate simulated vision to truly aid the visually impaired.

In the early 1930's, Foerster investigated the effect of electrically stimulating the exposed occipital pole of one cerebral hemisphere. He found that, when a point at the extreme occipital pole was stimulated, the patient perceived a small spot of light directly in front and motionless (a phosphene). Subsequently, Brindley and Lewin (1968) thoroughly studied electrical stimulation of the human occipital (visual) cortex. By varying the stimulation parameters, these investigators described in detail the location of the phosphenes produced relative to the specific region of the occipital cortex stimulated. These experiments demonstrated: (1) the consistent shape and position of phosphenes; (2) that increased stimulation pulse duration made phosphenes brighter; and (3) that there was no detectable interaction between neighboring electrodes which were as close as 2.4 mm apart.

As intraocular surgical techniques have advanced, it has become possible to apply stimulation on small groups and even on individual retinal cells to generate focused phosphenes through devices implanted within the eye itself. This has sparked renewed interest in developing methods and apparatuses to aid the visually impaired. Specifically, great effort has been expended in the area of intraocular visual prosthesis devices in an effort to restore vision in cases where blindness is caused by photoreceptor degenerative retinal diseases such as retinitis pigmentosa and age related macular degeneration which affect millions of people worldwide.

Neural tissue can be artificially stimulated and activated by prosthetic devices that pass pulses of electrical current through electrodes on such a device. The passage of current causes changes in electrical potentials across visual neuronal membranes, which can initiate visual neuron action potentials, which are the means of information transfer in the nervous system.

Based on this mechanism, it is possible to input information into the nervous system by coding the information as a sequence of electrical pulses which are relayed to the nervous system via the prosthetic device. In this way, it is possible to provide artificial sensations including vision.

One typical application of neural tissue stimulation is in the rehabilitation of the blind. Some forms of blindness involve selective loss of the light sensitive transducers of the retina. Other retinal neurons remain viable, however, and may be activated in the manner described above by placement of a prosthetic electrode device on the inner (toward the vitreous) retinal surface (epiretial). This placement must be mechanically stable, minimize the distance between the device electrodes and the visual neurons, and avoid undue compression of the visual neurons.

In 1986, Bullara (US Pat. No. 4,573,481) patented an electrode assembly for surgical implantation on a nerve. The matrix was silicone with embedded iridium electrodes. The assembly fit around a nerve to stimulate it.

Dawson and Radtke stimulated cat's retina by direct electrical stimulation of the retinal ganglion cell layer. These experimenters placed nine and then fourteen electrodes upon the inner retinal layer (i.e., primarily the ganglion cell layer) of two cats. Their experiments suggested that electrical stimulation of the retina with 30 to 100 uA current resulted in visual cortical responses. These experiments were carried out with needle-shaped electrodes that penetrated the surface of the retina (see also US Pat. No. 4,628,933 to Michelson).

The Michelson '933 apparatus includes an array of photosensitive devices on its surface that are connected to a plurality of electrodes positioned on the opposite surface of the device to stimulate the retina. These electrodes are disposed to form an array similar to a "bed of nails" having conductors which impinge directly on the retina to stimulate the retinal cells. US Patents 4,837,049 to Byers describes spike electrodes for neural stimulation. Each spike electrode pierces neural tissue for better electrical contact. US Patent 5,215,088 to Norman describes an array of spike electrodes for cortical stimulation. Each spike pierces cortical tissue for better electrical contact. US 2006/058857 describes an example of an implanted artificial vision system.

WO 96/01665 describes a patient programmer for implantable tissue stimulation.

The art of implanting an intraocular prosthetic device to electrically stimulate the retina was advanced with the introduction of retinal tacks in retinal surgery. De Juan, et al. at Duke University Eye Center inserted retinal tacks into retinas in an effort to reattach retinas that had detached from the underlying choroid, which is the source of blood supply for the outer retina and thus the photoreceptors. See, e.g., E. de Juan, et al., 99 Am. J. Ophthalmol. 272 (1985). These retinal tacks have proved to be biocompatible and remain embedded in the retina, and choroid/sclera, effectively pinning the retina against the choroid and the posterior aspects of the globe. Retinal tacks are one way to attach a retinal array to the retina. US Patent 5,109,844 to de Juan describes a flat electrode array placed against the retina for visual stimulation. US Patent 5,935,155 to Humayun describes a visual prosthesis for use with the flat retinal array described in de Juan.

### SUMMARY

The object of the present invention is achieved with a visual prosthesis apparatus according to claim 1.

Further embodiments are shown in the specification, drawings and claims of the present application.

### BRIEF DESCRIPTION OF THE FIGURES

Figure 1 shows a visual prosthesis apparatus according to the present disclosure.
Figures 2 and 3 show a retinal stimulation system adapted to be implanted into a subject.
Figure 4 shows a front view of the implanted retinal stimulation system.
Figure 5 shows a side view of the implanted system of Figure 9.
Figures 6A and 7 show a video capture/transmission apparatus or visor adapted to be used in combination with the retinal stimulation system of Figures 2-5.
Figures 6B shows exploded view of the external coil arrangement and mounting system shown in
Figures 6A and 7.

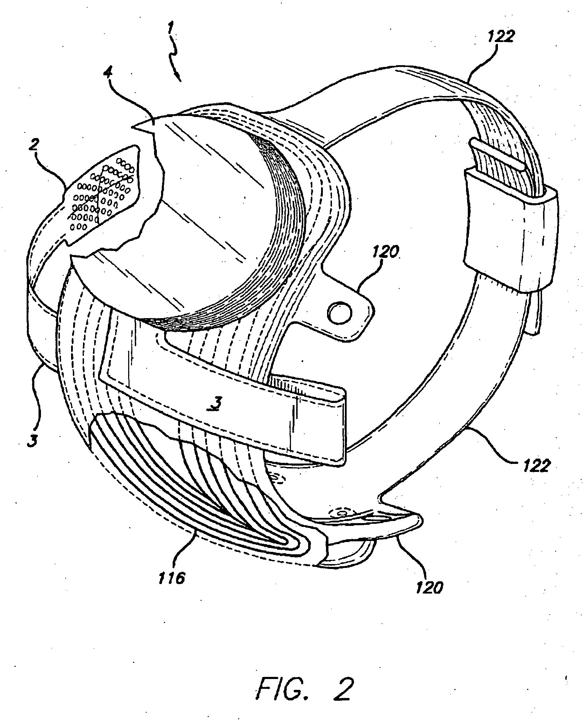

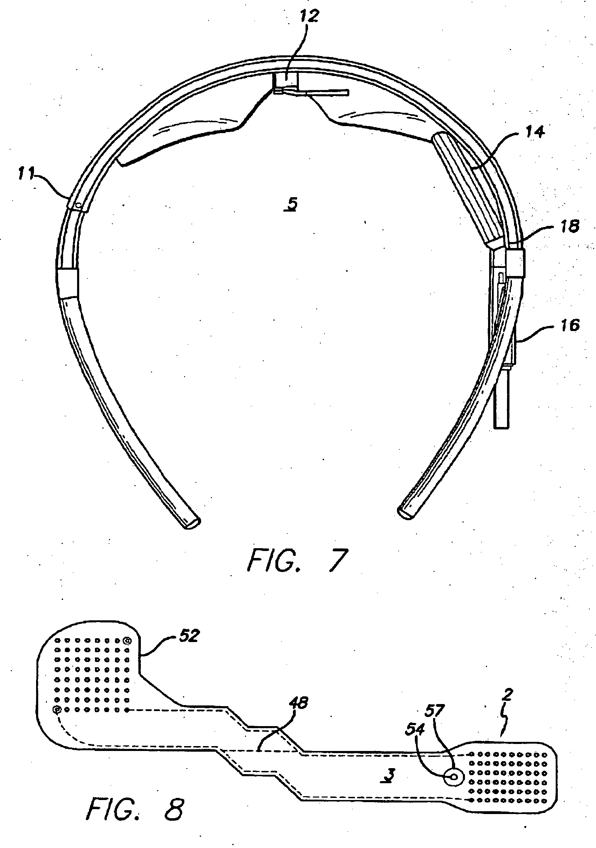

Figure 8 shows a flexible circuit electrode array, also shown in Figure 2.

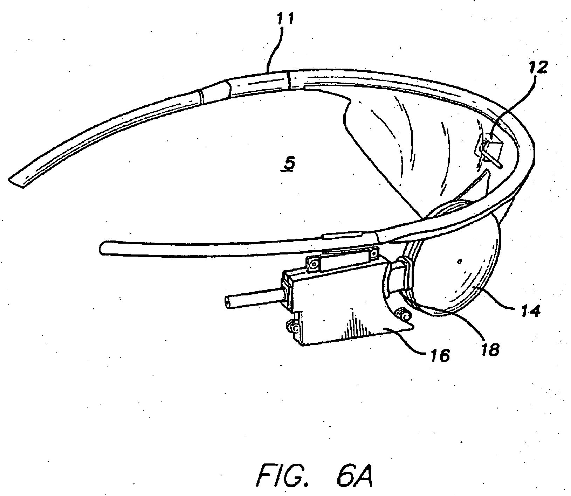

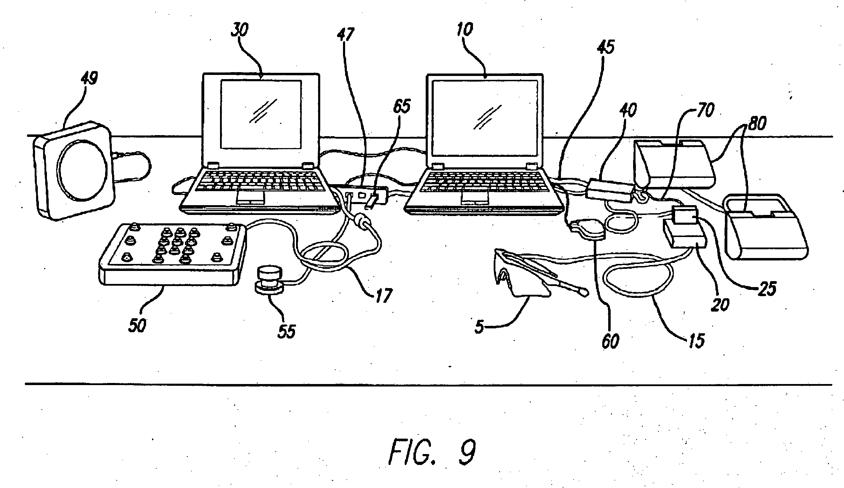

Figure 9 shows components of a fitting system according to the present disclosure, the system also comprising the visor shown in Figures 6A-6B and 7.

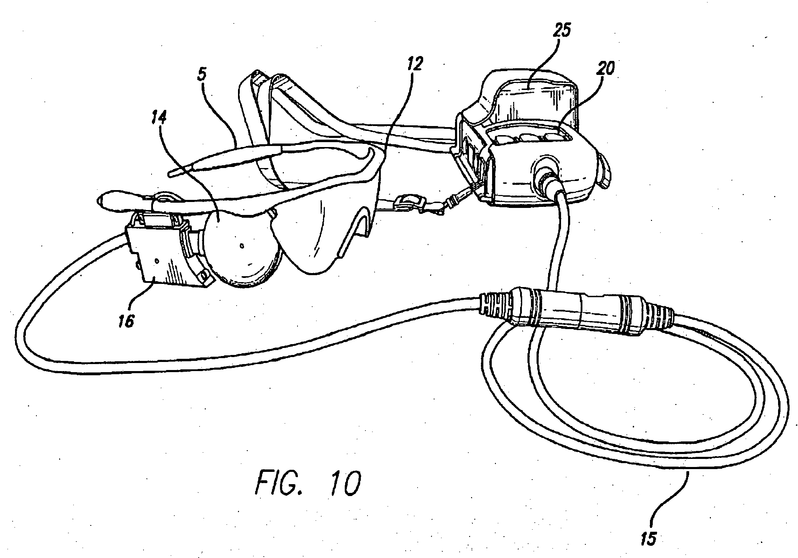

Figure 10 shows the visual prosthesis apparatus in a stand-alone mode, i.e. comprising the visor connected to a video processing unit.

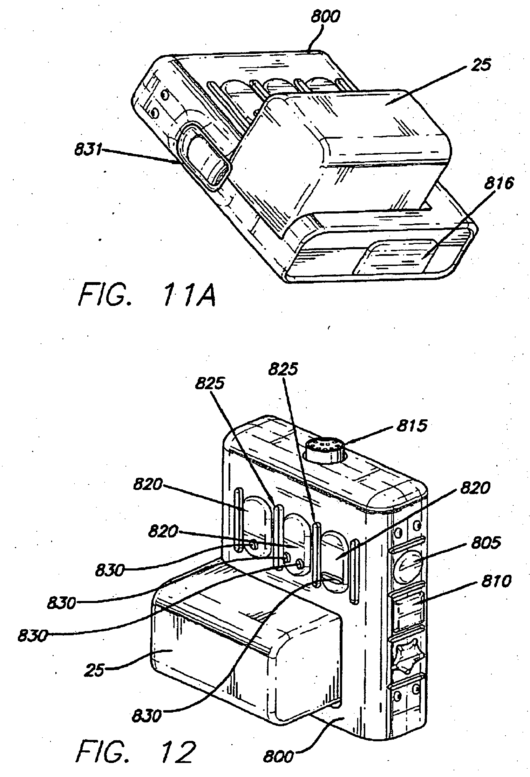

Figures 11A-G and 12 show the video processing unit already briefly shown with reference to Figures 9 and 10.

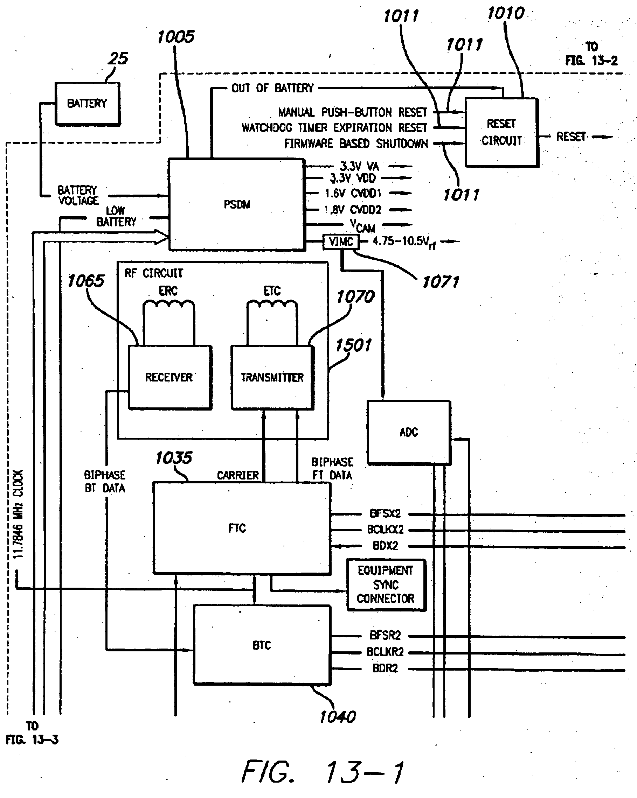

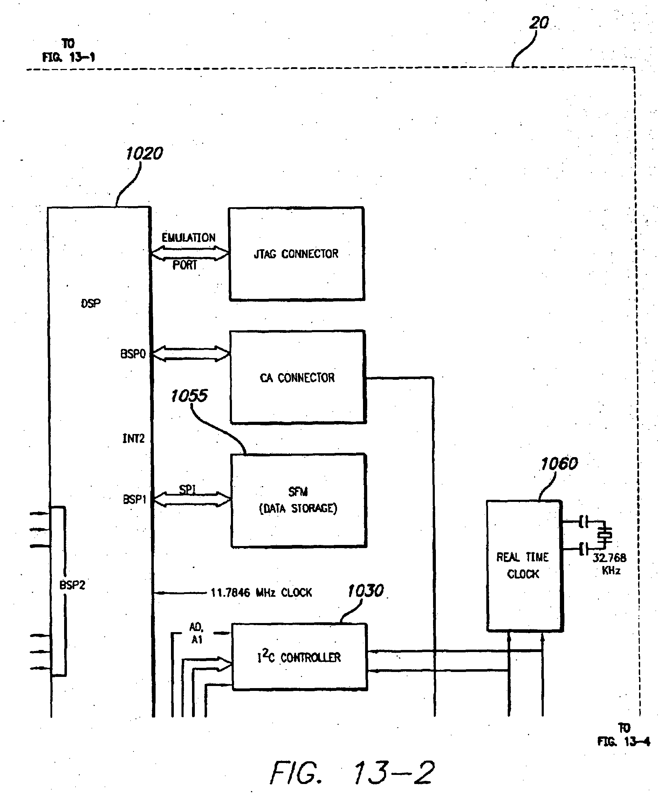

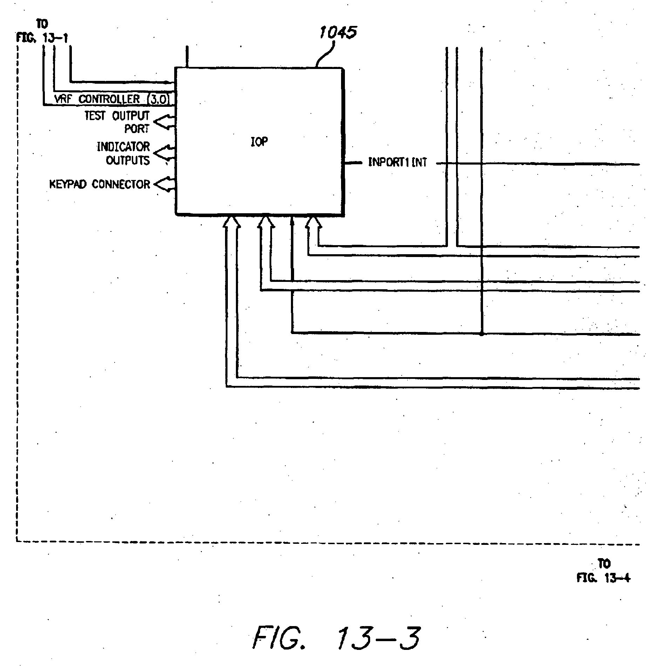

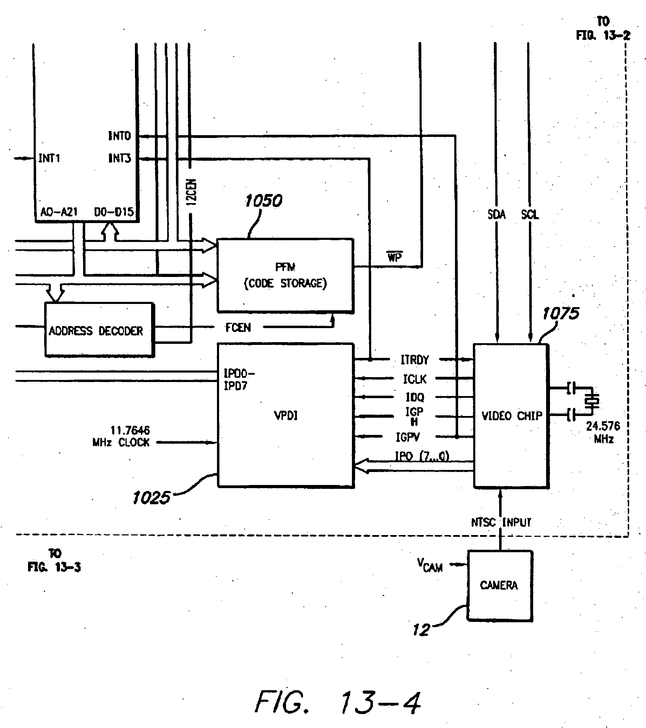

Figures 13-1, 13-2, 13-3 and 13-4 show an exemplary embodiment of a video processing unit. Figure 13-1 should be viewed at the left of Figure 13-2. Figure 13-3 should be viewed at the left of Figure 13-4. Figures 13-1 and 13-2 should be viewed on top of Figures 13-3 and 13-4.

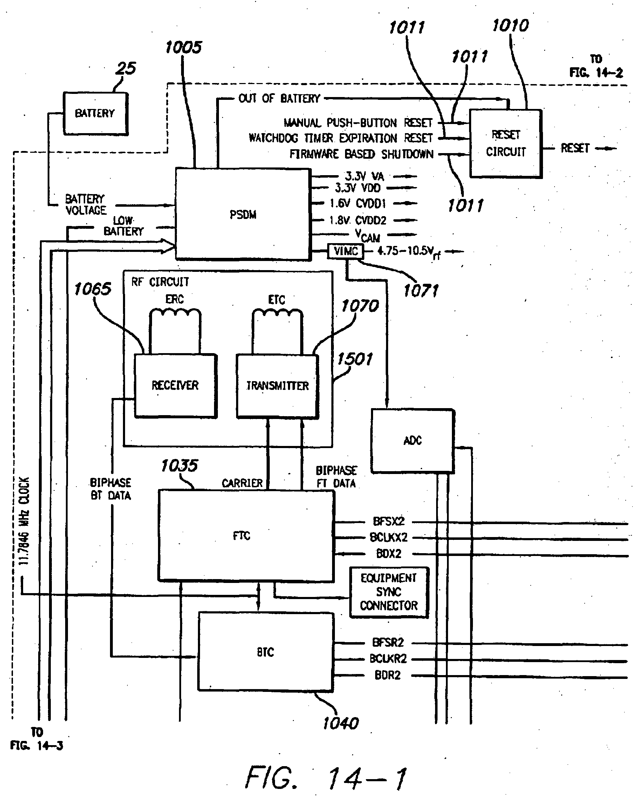

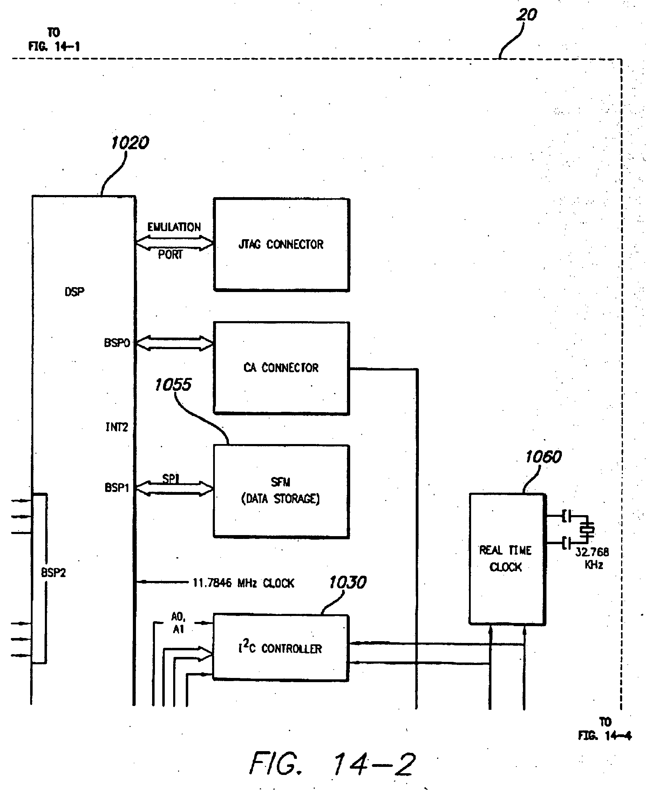

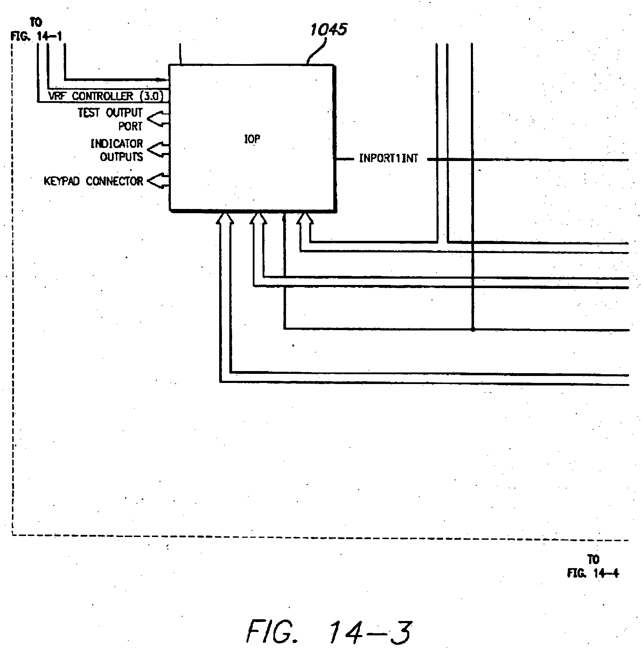

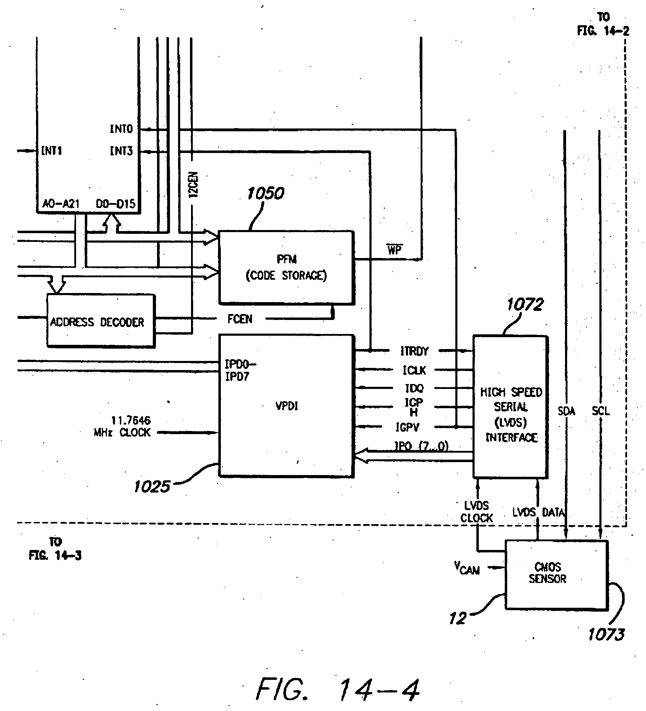

Figures 14-1, 14-2, 14-3 and 14-4 show another exemplary embodiment of a video processing unit. Figure 14-1 should be viewed at the left of Figure 14-2. Figure 14-3 should be viewed at the left of Figure 14-4. Figures 14-1 and 14-2 should be viewed on top of Figures 14-3 and 14-4.

In the following description, like reference numbers are used to identify like elements. Furthermore, the drawings are intended to illustrate major features of exemplary embodiments in a diagrammatic manner. The drawings are not intended to depict every feature of every implementation nor relative dimensions of the depicted elements, and are not drawn to scale.

### DETAILED DESCRIPTION

The present disclosure is concerned with a visual apparatus for creation of artificial vision. In particular, the present disclosure provides an interface and method for controlling a visual prosthesis (i.e. device) implanted in an individual patient (i.e. subject) to create artificial vision.

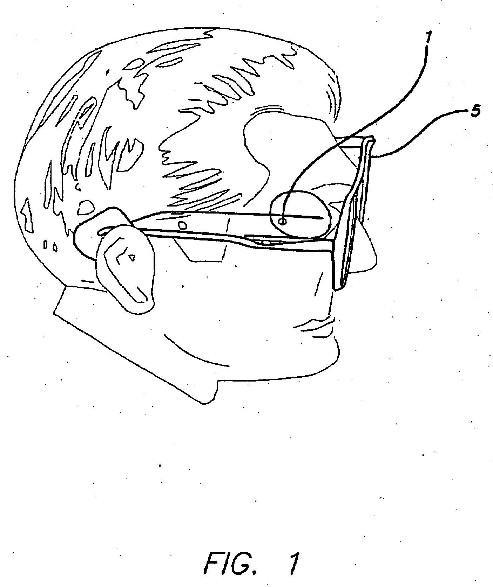

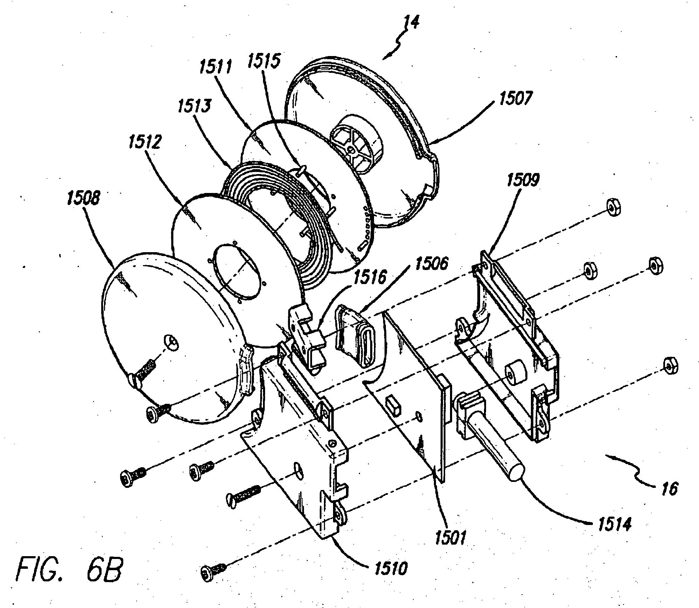

Figure 1 shows a visual prosthesis apparatus. The visual apparatus comprises, in combination, an implantable retinal stimulation system **1** and a video capture/transmission apparatus or visor embodied in visor/Glasses **5.** An exemplary retinal stimulation system **1** is shown in more detail in **Figures 2-5** and an exemplary visor **5** is shown in more detail in Figures **6** and **7****.**

The retinal stimulation system **1** is further disclosed in U.S. patent Application US 2006/0247754, filed August 19, 2005 for "Flexible Circuit Electrode Array" by Robert J. Greenberg, et, al. and is intended for use in subjects with retinitis pigmentosa. The visor **5** is further disclosed in International Patent Pulication No. WO 2007/149291, filed on June 14, 2007 and entitled "APPARATUS AND METHOD FOR ELECTRICAL STIMULATION OF HUMAN RETINA,".

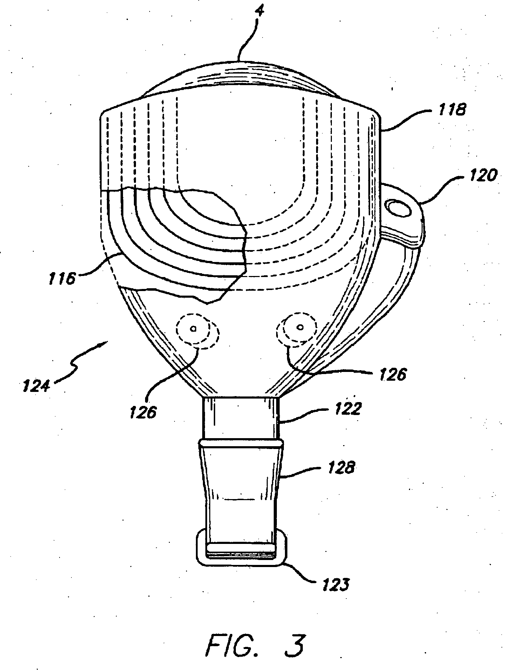

The exemplary retinal stimulation system **1,** shown in **Figures 2** and **3****,** is an implantable electronic device containing an inductive coil **116** and an electrode array **2** that is electrically coupled by a cable 3 that pierces sclera of the subject's eye to an electronics package **4,** external to the sclera. The retinal stimulation system **1** is designed, for example, to elicit visual percepts in blind subjects with retinitis pigmentosa.

Human vision provides a field of view that is wider than it is high. This is partially due to fact that we have two eyes, but even a single eye provides a field of view that is approximately 90° high and 140° to 160° degrees wide. It is therefore, advantageous to provide a flexible circuit electrode array **2** that is wider than it is tall. This is equally applicable to a cortical visual array. In which case, the wider dimension is not horizontal on the visual cortex, but corresponds to horizontal in the visual scene.

**Figure 8** shows the flexible circuit electrode array **2** prior to folding and attaching to the electronics package **4** of **Figure 2****.** At one end of the flexible circuit cable **3** is an interconnection pad **52** for connection to the electronics package **4.** At the other end of the flexible circuit cable **3** is the flexible circuit electrode array **2.** Further, an attachment point **54** may be provided near the flexible circuit electrode array **2.** A retina tack (not shown) is placed through the attachment point **54** to hold the flexible circuit electrode array **2** to the retina. A stress relief **57** may be provided surrounding the attachment point **54.** The stress relief **57** may be made of a softer polymer than the flexible circuit, or it may include cutouts or thinning of the polymer to reduce the stress transmitted from the retina tack to the flexible circuit electrode array **2.** The flexible circuit cable **3** may be formed in a dog leg pattern so than when it is folded at fold **48 it** effectively forms a straight flexible circuit cable 3 with a narrower portion at the fold **48** for passing through the sclerotomy. The electrode array 2 may comprise a polyimide cable that houses wire conductors and an array of exposed platinum electrodes in a grid. In one embodiment, there are sixty electrodes arranged in a 6x10 grid.

The electronics package **4** of **Figures 2** and **3** can be electrically coupled to the inductive coil **116.** In one aspect, the inductive coil **116** contains a receiver and transmitter antennae made from wound wire. Alternatively, the inductive coil **116** may be made from a thin film polymer sandwich with wire traces deposited between layers of thin film polymer. The electronics package **4** may contain components and an Application Specific Integrated Circuit (ASIC) for processing the received data and using the received power to generate the required stimulation output. The electronics package **4** and the inductive coil **116** may be held together by a molded body **118** shown in **Figure 3****.** As also shown in **Figure 3****,** the molded body **118** may also include suture tabs **120** shown in **Figure 3****.** The molded body narrows to form a strap **122** which surrounds the sclera and holds the molded body **118,** inductive coil **116,** and electronics package **4** in place. The molded body **118,** suture tabs **120** and strap **122** are preferably an integrated unit made of silicone elastomer. Silicone elastomer can be formed in a pre-curved shape to match the curvature of a typical sclera. Furthermore, silicone remains flexible enough to accommodate implantation and to adapt to variations in the curvature of an individual sclera. In one aspect, the inductive coil **116** and molded body **118** are oval shaped, and in this way, a strap **122** can better support the oval shaped coil.

The eye moves constantly. The eye moves to scan a scene and also has a jitter motion to prevent image stabilization. Even though such motion is useless in the blind, it often continues long after a person has lost their sight. Thus, in one embodiment of the present disclosure, the entire retinal stimulation system **1** of the prosthesis is attached to and supported by the sclera of a subject. By placing the device under the rectus muscles with the electronics package in an area of fatty tissue between the rectus muscles, eye motion does not cause any flexing which might fatigue, and eventually damage, the device.

**Figure 3** shows a side view of the retinal stimulation system **1,** in particular, emphasizing the fan tail **124.** When the retinal prosthesis is implanted, the strap **122** is passed under the eye muscles to surround the sclera. The inductive coil **116** and molded body **118** should also follow the strap under the lateral rectus muscle on the side of the sclera. The retinal stimulation system **1** of the visual prosthesis apparatus is very delicate. It is easy to tear the molded body **118** or break wires in the inductive coil **116.** In order to allow the molded body **118** to slide smoothly under the lateral rectus muscle, the molded body is shaped in the form of a fan tail **124** on the end opposite the electronics package **4.** Element **123** shows a retention sleeve, while elements **126** and **128** show holes for surgical positioning and a ramp for surgical positioning, respectively.

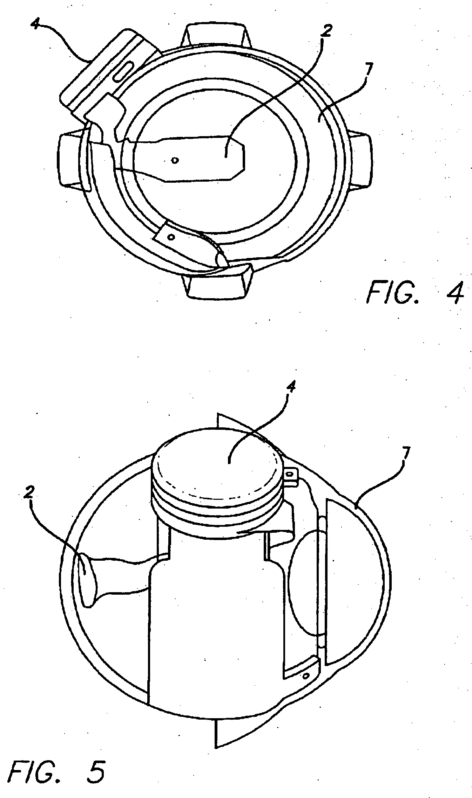

**Figures 4 and 5** show front and side views of the Retinal stimulation system **1** implanted with respect to the subject's eye 7. As shown in **Figures 4** and **5****,** the electrode array **2** enters the eye through a pars plana incision and is placed on the retina over the fovea using a retinal tack. The remaining Retinal stimulation system **1** is secured to the eye by means of a scleral band held in place by a Watzke sleeve (typical of scleral procedures), and also by suture tabs. Additionally, another suture may be placed around the scleral band in the inferior medical quadrant of the eye.

Referring to **Figures 6A** and **7****,** the glasses **5** may comprise, for example, a frame **11** holding a camera **12,** an external coil **14** and a mounting system **16** for the external coil **14.** The mounting system **16** may also enclose the RF circuitry. In this configuration, the video camera **12** captures live video. The video signal is sent to an external Video Processing Unit (VPU) **20** (shown in **Figures 9-12** and discussed below), which processes the video signal and subsequently transforms the processed video signal into electrical stimulation patterns or data. The electrical stimulation data are then sent to the external coil **14** that sends both data and power via radio-frequency (RF) telemetry to the coil **116** of the retinal stimulation system **1,** shown in **Figures 2** and **3****.** The coil **116** receives the RF commands which control the application specific integrated circuit (ASIC) which in turn delivers stimulation to the retina of the subject via a thin film electrode array (TFEA). In one aspect of an embodiment, light amplitude is recorded by the camera **12.** The VPU **20** may use a logarithmic encoding scheme to convert the incoming light amplitudes into the electrical stimulation patterns or data. These electrical stimulation patterns or data may then be passed on to the Retinal Stimulation System 1, which results in the retinal cells being stimulated via the electrodes in the electrode array 2 (shown in **Figures 2****,** **3** and **8****).** In one exemplary embodiment, the electrical stimulation patterns or data being transmitted by the external coil **14** is binary data. The external coil **14** may contain a receiver and transmitter antennae and a radio-frequency (RF) electronics card for communicating with the internal coil **116.**

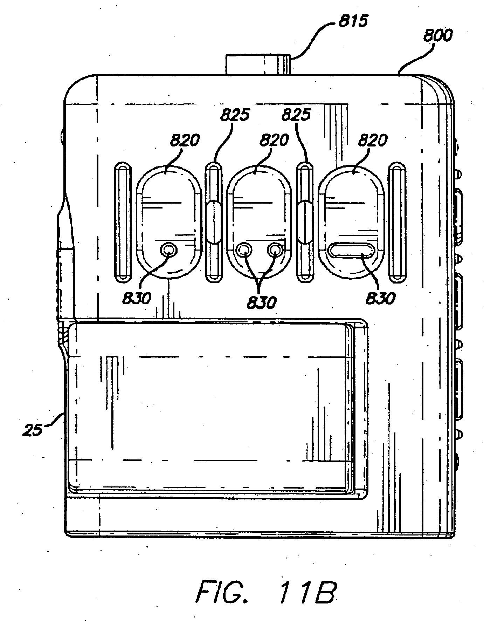

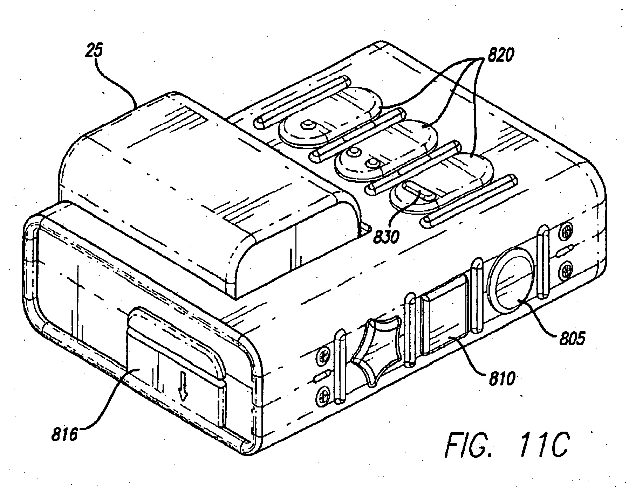

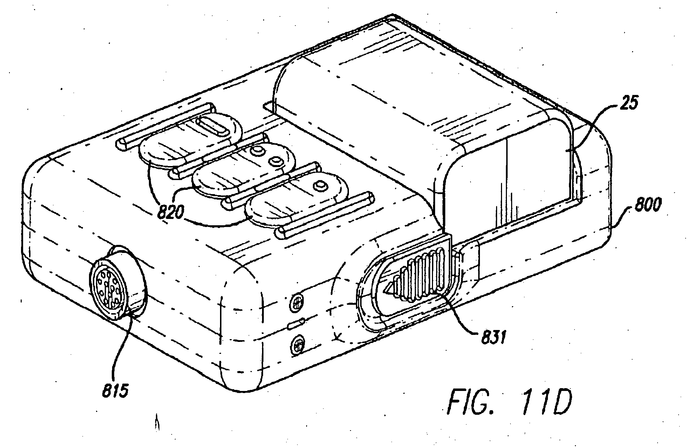

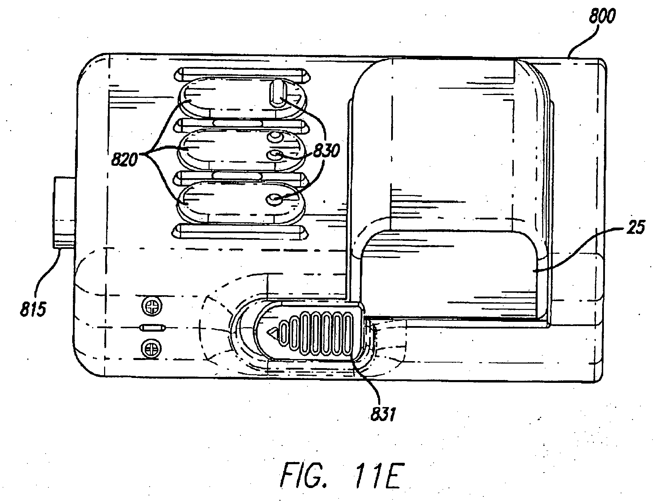

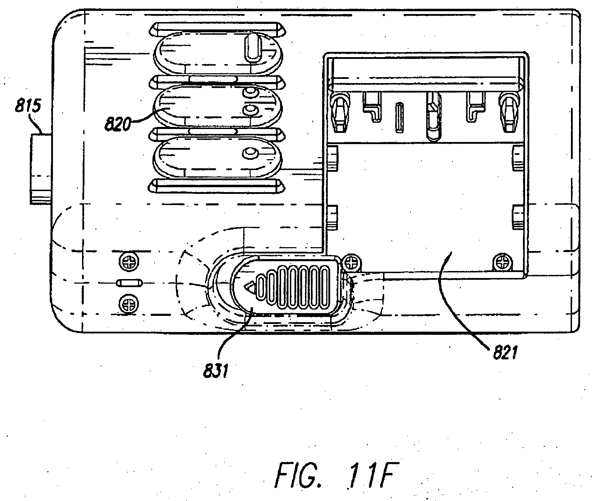

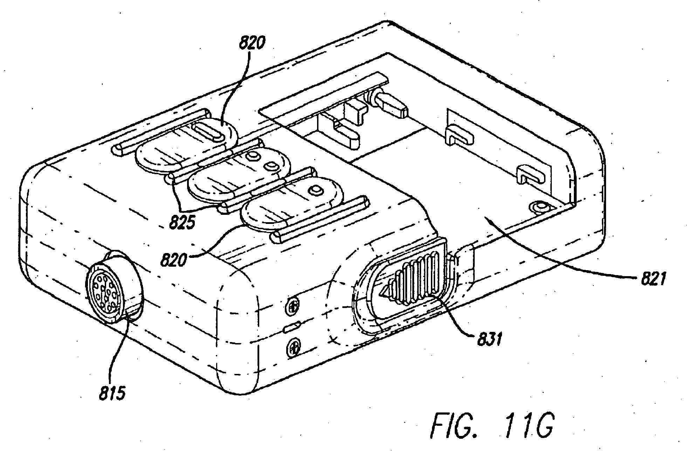

**Figure 6B** shows an exploded view of the external coil arrangement **14** and mounting system **16.** As also shown in **Figures 6A** and **7****,** the external coil arrangement **14** and mounting system **16** are connected by a flexible connector **1506.** In particular, the flexible connector **1506** is attached to RF coil enclosure halves **1507** and **1508** on the coil side and to enclosure case halves **1509** and **1510** on the mounting system side. The external coil arrangement **14** comprises coil enclosure halves **1507** and **1508,** enclosing printed circuit boards (PCB) **1511** and **1512** surrounding an RF transmitting coil **1513.** The PCBs **1511** and **1512** may further include telemetry receiving coils. The mounting system **16** comprises case halves **1509** and **1510** enclosing an RF visor cable assembly **1514.** Other mechanical components shown in **Figure 6B** include: wires **1515** connecting PCBs **1511** and **1512;** a mounting bracket **1516;** and RF circuitry **1501** located between case halves **1509** and **1510.** While video image processing is done in a VPU **20** (shown in **Figures 11-12****),** the RF circuitry **1501** is incorporated into the mounting system **16** to reduce losses in the cable connecting the VPU **20** to the Glasses 5. PCBs **1511** and **1512** can be made of glass base epoxy and laminated with copper. An exemplary circuital diagram of RF circuitry **1501** is shown in **Figures 13-1** to **13-4.**

Referring to **Figure 9****,** a Fitting System (FS) may be used to configure and optimize the visual prosthesis apparatus shown in **Figure 1****.** The Fitting System is fully described in the related application U.S. Patent No. US 2007/0255343, filed on April 27, 2007.

The Fitting System may comprise custom software with a graphical user interface running on a dedicated laptop computer **10.** Within the Fitting System are modules for performing diagnostic checks of the implant, loading and executing video configuration files, viewing electrode voltage waveforms, and aiding in conducting psychophysical experiments. A video module can be used to download a video configuration file to the Video Processing Unit (VPU) **20** discussed above and store it in non-volatile memory to control various aspects of video configuration, e.g. the spatial relationship between the video input and the electrodes. The software can also load a previously used video configuration file from the VPU 20 for adjustment.

The Fitting System can be connected to the Psychophysical Test System (PTS), located for example on a dedicated laptop **30,** in order to run psychophysical experiments. In psychophysics mode, the Fitting System enables individual electrode control, permitting clinicians to construct test stimuli with control over current amplitude, pulse-width, and frequency of the stimulation. In addition, the psychophysics module allows the clinician to record subject responses. The PTS may include a collection of standard psychophysics experiments developed using for example MATLAB (Math Works) software and other tools to allow the clinicians to develop customized psychophysics experiment scripts.

Using the psychophysics module, important perceptual parameters such as perceptual threshold, maximum comfort level, and spatial location of percepts may be reliably measured. Based on these perceptual parameters, the fitting software enables custom configuration of the transformation between video image and spatio-temporal electrode stimulation parameters in an effort to optimize the effectiveness of the retinal prosthesis for each subject.

The Fitting System laptop **10** of **Figure 9** may be connected to the VPU **20** using an optically isolated serial connection adapter **40.** Because it is optically isolated, the serial connection adapter **40** assures that no electric leakage current can flow from the Fitting System laptop **10 in** the even of a fault condition.

As shown in **Figure 9****,** the following components may be used with the Fitting System according to the present disclosure. The Video Processing Unit (VPU) **20** for the subject being tested, a Charged Battery **25** for VPU **20,** the Glasses **5,** a Fitting System (FS) Laptop **10,** a Psychophysical Test System (PTS) Laptop **30,** a PTS CD (not shown), a Communication Adapter (CA) **40,** a USB Drive (Security) (not shown), a USB Drive (Transfer) **47,** a USB Drive (Video Settings) (not shown), a Patient Input Device (RF Tablet) **50,** a further Patient Input Device (Jog Dial) **55,** Glasses Cable **15,** CA-VPU Cable **70,** FS-CA Cable **45,** FS-PTS Cable **46,** Four (4) Port USB Hub **47,** Mouse **60,** Test Array system **80,** Archival USB Drive **49,** an Isolation Transformer (not shown), adapter cables (not shown), and an External Monitor (not shown).

With continued reference to **Figure 9****,** the external components of the Fitting System may be configured as follows. The battery **25** is connected with the VPU **20.** The PTS Laptop **30** is connected to FS Laptop **10** using the FS-PTS Cable **46.** The PTS Laptop **30** and FS Laptop **10** are plugged into the Isolation Transformer (not shown) using the Adapter Cables (not shown). The Isolation Transformer is plugged into the wall outlet. The four (4) Port USB Hub **47** is connected to the FS laptop **10** at the USB port. The mouse **60** and the two Patient Input Devices **50** and **55** are connected to four (4) Port USB Hubs **47.** The FS laptop **10** is connected to the Communication Adapter (CA) **40** using the FS-CA Cable **45.** The CA **40** is connected to the VPU **20** using the CA-VPU Cable **70.** The Glasses **5** are connected to the VPU **20** using the Glasses Cable **15.**

In one exemplary embodiment, the Fitting System shown in **Figure 9** may be used to configure system stimulation parameters and video processing strategies for each subject outfitted with the visual prosthesis apparatus of **Figure 1****.** The fitting application, operating system, laptops **10** and **30,** isolation unit and VPU **20** may be tested and configuration controlled as a system. The software provides modules for electrode control, allowing an interactive construction of test stimuli with control over amplitude, pulse width, and frequency of the stimulation waveform of each electrode in the Retinal stimulation system **1.** These parameters are checked to ensure that maximum charge per phase limits, charge balance, and power limitations are met before the test stimuli are presented to the subject. Additionally, these parameters may be checked a second time by the VPU **20'**s firmware. The Fitting System shown in **Figure 7** may also provide a psychophysics module for administering a series of previously determined test stimuli to record subject's responses. These responses may be indicated by a keypad **50** and or verbally. The psychophysics module may also be used to reliably measure perceptual parameters such as perceptual threshold, maximum comfort level, and spatial location of percepts. These perceptual parameters may be used to custom configure the transformation between the video image and spatio-tempral electrode stimulation parameters thereby optimizing the effectiveness of the visual prosthesis for each subject. The Fitting System is fully described in the related application U.S. Patent Application No. US 2007/0255343, filed on April 27, 2007.

The visual prosthesis apparatus of **Figure 1** may operate in two modes: i) stand-alone mode and ii) communication mode

### Stand-Alone Mode

Referring to **Figures 1****,** **2** and **10****,** in the stand-alone mode, the video **camera 12,** on the glasses **5,** captures a video image that is sent to the VPU **20.** The VPU 20 processes the image from the camera **12** and transforms it into electrical stimulation patterns that are transmitted to the external coil **14.** The external coil **14** sends the electrical stimulation patterns and power via radio-frequency (RF) telemetry to the implanted retinal stimulation system **1 (****Figures 2** and **3****).** The internal coil **116** of the retinal stimulation system **1** receives the RF commands from the external coil **14** and transmits them to the electronics package **4** that in turn delivers stimulation to the retina via the electrode array **2.** Additionally, the retinal stimulation system **1** may communicate safety and operational status back to the VPU **20** by transmitting RF telemetry from the internal coil **116** to the external coil **14.** The visual prosthesis apparatus of **Figure 1** may be configured to electrically activate the retinal stimulation system **1** only when it is powered by the VPU **20** through the external coil **14.** The stand-alone mode may be used for clinical testing and/or at-home use by the subject.

### Communication Mode

The communication mode may be used for diagnostic testing, psychophysical testing, patient fitting and downloading of stimulation settings to the VPU **20** before transmitting data from the VPU **20** to the retinal stimulation system **1** as is done for example in the stand-alone mode described above. Referring to **Figure 9****,** in the communication mode, the VPU **20** is connected to the Fitting System laptop **10** using cables **70, 45** and the optically isolated serial connection adapter **40.** In this mode, laptop **10** generated stimuli may be presented to the subject and programming parameters may be adjusted and downloaded to the VPU **20.** The Psychophysical Test System (PTS) laptop **30** connected to the Fitting System laptop **10** may also be utilized to perform more sophisticated testing and analysis as fully described in the related application U.S. Application No. 11/796,425, filed on April 27, 2007, (Applicant's Docket No. S401-USA) which is incorporated herein by reference in its entirety.

In one embodiment, the functionality of the retinal stimulation system **1** can also be tested pre-operatively and intra-operatively (i.e. before operation and during operation) by using an external coil **14,** without the glasses **5,** placed in close proximity to the retinal stimulation system **1.** The coil **14** may communicate the status of the retinal stimulation system **1** to the VPU **20** that is connected to the Fitting System laptop **10** as shown in **Figure 9****.**

As discussed above, the VPU **20** processes the image from the camera **12** and transforms the image into electrical stimulation patterns for the retinal stimulation system **1.** Filters such as edge detection filters may be applied to the electrical stimulation patterns for example by the VPU **20** to generate, for example, a stimulation pattern based on filtered video data that the VPU **20** turns into stimulation data for the retinal stimulation system **1.** The images may then be reduced in resolution using a downscaling filter. In one exemplary embodiment, the resolution of the image may be reduced to match the number of electrodes in the electrode array **2** of the retinal stimulation system **1.** That is, if the electrode array has, for example, sixty electrodes, the image may be reduced to a sixty channel resolution. After the reduction in resolution, the image is mapped to stimulation intensity using for example a look-up table that has been derived from testing of individual subjects. Then, the VPU **20** transmits the stimulation parameters via forward telemetry to the retinal stimulation system **1** in frames that may employ a cyclic redundancy check (CRC) error detection scheme.

In one exemplary embodiment, the VPU **20** may be configured to allow the subject/patient i) to turn the visual prosthesis apparatus on and off, ii) to manually adjust settings, and iii) to provide power and data to the retinal stimulation system **1.** Referring to **Figures 11** and **12****,** the VPU **20** may comprise a case **800,** power button **805** for turning the VPU **20** on and off, setting button **810,** zoom buttons **820** for controlling the camera **12,** connector port **815** for connecting to the Glasses **5,** a connector port **816** for connecting to the laptop **10** through the connection adapter **40,** indicator lights **825** to give visual indication of operating status of the system, the rechargeable battery **25** for powering the VPU **20,** battery latch **831** for locking the battery **25** in the case **800,** digital circuit boards (not shown), and a speaker (not shown) to provide audible alerts to indicate various operational conditions of the system. Because the VPU **20** is used and operated by a person with minimal or no vision, the buttons on the VPU **20** may be differently shaped and/or have special markings as shown in **Figure 12** to help the user identify the functionality of the button without having to look at it. As shown in Figure **12****,** the power button **805** may be a circular shape while the settings button **820** may be square shape and the zoom buttons **820** may have special raised markings **830** to also identify each buttons functionality. One skilled in the art would appreciate that other shapes and markings can be used to identify the buttons without departing from the spirit and scope of the invention. For example, the markings can be recessed instead of raised.

In one embodiment, the indicator lights **825** may indicate that the VPU **20** is going through system start-up diagnostic testing when the one or more indicator lights **825** are blinking fast (more then once per second) and are green in color. The indicator lights **825** may indicate that the VPU **20** is operating normally when the one or more indicator lights **825** are blinking once per second and are green in color. The indicator lights **825** may indicate that the retinal stimulation system **1** has a problem that was detected by the VPU **20** at start-up diagnostic when the one or more indicator lights **825** are blinking for example once per five second and are green in color. The indicator lights **825** may indicate that the video signal from camera **12** is not being received by the VPU **20** when the one or more indicator lights **825** are always on and are amber color. The indicator lights **825** may indicate that there is a loss of communication between the retinal stimulation system **1** and the external coil **14** due to the movement or removal of Glasses **5** while the system is operational or if the VPU **20** detects a problem with the retinal stimulation system **1** and shuts off power to the retinal stimulation system **1** when the one or more indicator lights **825** are always on and are orange color. One skilled in the art would appreciate that other colors and blinking patterns can be used to give visual indication of operating status of the system without departing from the spirit and scope of the invention.

In one embodiment, a single short beep from the speaker (not shown) may be used to indicate that one of the buttons **825, 805** or **810** have been pressed. A single beep followed by two more beeps from the speaker (not shown) may be used to indicate that VPU **20** is turned off. Two beeps from the speaker (not shown) may be used to indicate that VPU **20** is starting up. Three beeps from the speaker (not shown) may be used to indicate that an error has occurred and the VPU **20** is about to shut down automatically. As would be clear to one skilled in the are different periodic beeping may also be used to indicate a low battery voltage warning, that there is a problem with the video signal, and/or there is a loss of communication between the retinal stimulation system **1** and the external coil **14.** One skilled in the art would appreciate that other sounds can be used to give audio indication of operating status of the system without departing from the spirit and scope of the invention. For example, the beeps may be replaced by an actual prerecorded voice indicating operating status of the system.

In one exemplary embodiment, the VPU **20** is in constant communication with the retinal stimulation system **1** through forward and backward telemetry. In this document, the forward telemetry refers to transmission from VPU **20** to the retinal stimulation system **1** and the backward telemetry refers to transmissions from the Retinal stimulation system **1** to the VPU **20.** During the initial setup, the VPU **20** may transmit null frames (containing no stimulation information) until the VPU **20** synchronizes with the Retinal stimulation system **1** via the back telemetry. In one embodiment, an audio alarm may be used to indicate whenever the synchronization has been lost.

In order to supply power and data to the Retinal stimulation system **1,** the VPU **20** may drive the external coil **14** with a 3 MHz signal. To protect the subject, the retinal stimulation system **1** may comprise a failure detection circuit to detect direct current leakage and to notify the VPU **20** through back telemetry so that the visual prosthesis apparatus can be shut down.

One exemplary embodiment of the VPU **20** is shown in Figures **13-1****,** **13-2****,** **13-3** and **13-4****.** As shown in Figures **13-1****,** **13-2****,** **13-3** and **13-4****,** the VPU **20** may comprise: a Power Supply, a Distribution and Monitoring Circuit (PSDM) **1005,** a Reset Circuit **1010,** a System Main Clock (SMC) source (not shown), a Video Preprocessor Clock (VPC) source (not shown), a Digital Signal Processor (DSP) **1020,** Video Preprocessor Data Interface **1025,** a Video Preprocessor **1075,** an I²C Protocol Controller **1030,** a Complex Programmable Logic device (CPLD) (not shown), a Forward Telemetry Controller (FTC) **1035,** a Back Telemetry Controller (BTC) **1040,** Input/Output Ports **1045,** Memory Devices like a Parallel Flash Memory (PFM) **1050** and a Serial Flash Memory (SFM) **1055,** a Real Time Clock **1060,** an RF Voltage and Current Monitoring Circuit (VIMC) **1071,** a speaker and/or a buzzer (not shown), an RF receiver **1065,** and an RF transmitter **1070.**

In one exemplary embodiment, the VPU **20** is a battery **25** powered micro-controller-based video processing and radio-frequency (RF) transceiver system. The VPU **20** may be comprised of a digital circuit for processing video from camera **12** (see **Figure 10****)** and an RF circuit **1501,** shown in **Figure 13-1****,** to facilitate 2-way communication with the retinal stimulation system **1.** In one exemplary embodiment, the RF circuit **1501** may be mounted in the enclosure **1510** on the visor/Classes **5** in close proximity to the retinal stimulation system **1** while the digital circuit of the VPU **20** can be remotely located in a case **800** shown in **Figures 11A-E** and described below. The mechanical design, shown in **Figure 10****,** may include a multiple-conductor cable **15** linking the VPU **20** and RF circuits **1501** disposed in the mounting system **16** as shown in **Figures 6A** and **6B****.** In another exemplary embodiment, the RF circuits **1501** may further comprise all the elements shown in **Figures 13-1** to **13-4** that are disposed on the visor/Classes 5 in the mounting system **16.**

In an exemplary embodiment, the digital circuit of the VPU **20** accepts an incoming analog video stream from a small camera **12** mounted on the Glasses **5,** which may have the appearance of a pair of sunglasses. The analog video stream from the camera **12** is converted to a digital video stream by the Video Preprocessor **1075** of the VPU **20.** The digital video stream is then stored into memory as individual video frames typically by means of a direct memory access (DMA) circuit specifically designed to accept digital video, called the video preprocessor data interface (VDPI) **1025** shown in **Figure 13-4****.** The VDPI **1025** of the VPU **20** processes the video frames stored in its memory into packets of stimulation information. The stimulation information is passed to the RF circuit **1501** via a serial port and an encoder circuit within the forward telemetry controller (FTC) **1035.** The RF transmitter circuit **1070** receives the stimulation information and transmits it to the retinal stimulation system **1** via a magnetically coupled coil **14.** The RF signal within the coil **14** has sufficient strength to power the retinal stimulation system **1.** The RF circuit **1501** is also capable of receiving back-telemetry information (containing status and safety related data) from the retinal stimulation system 1 via a coil connected to a receiver circuit **1065.** The received data is passed to a decoder circuit within the back telemetry controller (BTC) **1040** and then to a serial port, which allows the VDPI **1025** to examine the received data. The power supply, distribution and monitoring circuit (PSDM) **1005** generates all of the appropriate voltages and contains a programmable power supply for the RF circuit **1501** to allow adjustment of the power level to the retinal stimulation system **1.**

The Power Supply, Distribution and Monitoring Circuit (PSDM) **1005** may regulate a variable battery voltage to several stable voltages that apply to components of the VPU 20. The Power Supply, Distribution and Monitoring Circuit (PSDM) **1005** may also provide low battery monitoring and depleted battery system cutoff. The PSDM **1005** may be configured to provide Digital Circuit Voltage **(VDD)** 3.3± 0.1 V@300mA; 300mV ripple. The PSDM **1005** may be configured to provide DSP Core Voltage **(CVDD1)** 1.6± 0.1V@100mA; 200mV ripple. The PSDM 1005 may be configured to provide CPLD Core Voltage **(CVDD2)** 1.8± 0.1V@50mA; 200mV ripple. The PSDM **1005** may be configured to provide Abalog Voltage **(VA)** 3.3± 0.1V@100mA; 300mV ripple. The PSDM **1005** may be configured to provide Camera Voltage (V_{CAM}) 5.0± 0.25V@100mA; 300mV ripple. The PSDM **1005** may be configured to provide RF Voltage 185mV/step (V_{rf}) 4.75V∼10.5V± 0.3V@75mA; 300mV ripple. The PSDM **1005** may be configured to provide Fixed Voltage (FV) 3.3± 0.1V@25mA; 100mV ripple. In one exemplary embodiment, the RF voltage (V_{rf}) may be fused on the RF circuit as a failsafe mechanism to protect against excessive current in case of a malfunction.

In one exemplary embodiment, the VPU **20** stops providing power/data to the retinal stimulation system **1** and/or turns itself off when the PSDM **1005** detects that the battery **25's (****Figure 10****)** voltage decreases to a predetermined voltage. The predetermined voltage may, for example, be 6.75± 0.4 volts. In one exemplary embodiment, the battery **25's** voltage may be fused at the battery connector (not shown) as a fail-safe mechanism to protect against excessive current in case of malfunction. In another exemplary embodiment, the battery **25's** drain current may be less than 1 mA when VPU **20** is turned off.

The Reset Circuit **1010** may have reset inputs **1011** that are able to invoke a system level reset. For example, the reset inputs **1011** may be from a manual push-button reset, a watchdog timer expiration, and/or firmware based shutdown. The manual push-button reset may be, for example, low active when pulse width is >1ms. The watchdog timer reset may be activated when the watchdog timer is not reset within a predetermined expiration time, wherein the watchdog timer should be reset every 1.0 seconds or less if the expiration time ranges, for example, from 1.0 to 2.25 seconds. The firmware-based shutdown may be activated when the firmware evokes, for example, a falling edge signal.

The System Main Clock (SMC) source is a clock source for DSP **1020** and CPLD. The SMC may be, for example, 11.7846 MHz +/- 50 ppm. The Video Preprocessor Clock (VPC) source is a clock source for the Video Processor and the VPC may be, for example, 24.576 MHz +/- 50 ppm.

The DSP **1020** may act as the central processing unit of the VPU 20. In one exemplary embodiment, the DSP **1020** is a Texas Instruments (TI) TMS 320VC5416PGE160. "TMS320VC5416PGE160 Fixed-Point Digital Signal Processor Data Manual" from TI is. The DSP **1020** may communicate with the rest of the components of the VPU **20** through parallel and serial interfaces. The parallel interface of the DSP **1020** may create a Program Space (PS), a Data Space (DS) and Input/Output Space (IOS), wherein the executable code is allocated to PS, the data is allocated to the DS and I/O devices are allocated to IOS. The serial interface DSP **1020** may contain three Multi-channel Buffered Serial Ports (McBSPs), wherein McBSP0 is configured in continuous clock mode for PC communication, McBSP1 is configured in non-continuous clock Serial Port Interface (SPI) mode for interface to the serial flash memory, and McBSP2 is configured in continuous clock mode for the RF transceiver.

The Video Processor **1075** may convert the NTSC signal from the camera **12** into a down-scaled resolution digital image format. In one exemplary embodiment, the Video Processor **1075** is a Philips Semiconductor SAA7114H. The Video Processor **1075** may comprise a video decoder (not shown) for converting the NTSC signal into high-resolution digitized image and a video scaler (not shown) for scaling down the high-resolution digitized image from the video decoder to an intermediate digitized image resolution. The video decoder of the Video Processor **1075** may be composed of an Analog Input Processing, Chrominance and Luminance Processing and Brightness Contrast and Saturation (BSC) Control circuits. The video scaler may be composed of Acquisition control, Pre-scaler, BSC-control, Line Buffer and Output Interface.

The I²C Protocol Controller **1030** may serve as a link between the DSP **1020** and the I²C bus. In one exemplary embodiment, the I²C Protocol Controller **1030** is a Philips Semiconductor PCA9564. The I²C Protocol Controller **1030** may be able to convert the parallel bus interface of the DSP **1020** to the I²C protocol bus or vise versa. The I²C Protocol Controller **1030** may also be connected to the Video Processor **1075** having, for example, a Read Address 43H and a Write Adress 42H and the I²C Protocol Controller **1030** may be connected to the Real Time Clock **1060** wherein the clock control registers use, for example, read address 0DFH and write address 0DEH and wherein the EEPROM array uses, for example, read address 0AFH and a write address 0AEH.

The Complex Programmable Logic Device (CPLD) (not shown) furnishes the physical device for the multiple digital logic circuits. Memory space allocation: the executable code may be mapped to parallel flash memory device that is located at off-DSP space (PS 00000H∼7FFFFH). The peripheral devices may be mapped to IOS as follows: Status/Timeput Port of the I²C communication device may be mapped to 2000H; Data Port of the I²C communication device may be mapped to 2001H; Own Address Port of the I²C communication device may be mapped to 2002H; and Control Port of the I²C communication device may be mapped to 2003H; Input port1 of the CPLD device may be mapped to 4000H; Inputport2 of the CPLD device may be mapped to 4001H; VideoDataInput of the CPLD device may be mapped to 4002H; CPLDVersion of the CPLD device may be mapped to 4003H; VideoLineCount of the CPLD device may be mapped to 4004H; OutputPort1 of the CPLD device may be mapped to 4008H; OutputPort2 of the CPLD device may be mapped to 400AH; OutputPort3 of the CPLD device may be mapped to 400BH; ADconvst of the CPLD device may be mapped to 6000H; ADCeN[1..0] of the CPLD device may be mapped to 8000∼8003H; and KeypadInput of the CPLD device may be mapped to A000H.

The VPDI **1025** may contain a tri-state machine to shift video data from Video Preprocessor **1075** to the DSP **1020.** Signal **ITRDY** of the VPDI **1025** may be connected to Video Chip **1075** and DSP **1020** and may be used to indicate the status of shifting and to facilitate rapid response in transferring video data. In one exemplary embodiment, a high level signal **ITRDY** indicates to VPU **20** that VPDI **1025** is empty and ready to accept next data, and a low level signal **ITRDY** indicates to the VPU **20** that VPDI **1025** is full and the Video Preprocessor **1075** should hold in the Video Preprocessor **1075's** internal FIFO (not shown). In one exemplary embodiment, the VPDI **1025** may have a coupling signal FreeVideo that enables or disables the VPDI **1025's** functionality.

The Forward Telemetry Controller (FTC) **1035** of **Figure 13-1** may pack 1024 bits of forward telemetry data into a forward telemetry frame. The FTC **1035** retrieves the forward telemetry data from the DSP **1020's** McBSP2 port and converts the data from logic level to biphase marked data. The FTC **1035** may generate **BCLKX2** and **BFSX2** signals to the DSP **1020's** MCBSP2 port. In one exemplary embodiment, the **BCLKX2** signal may be a serial data clock or forward telemetry data and the **BFSX2** signal may be an FX frame synchronization signal that may indicate that a first bit of each 16-bit word is generated by the serial part (MCBSP2). To improve reliability of data transmission, the FTC **1035** may also convert a logic '1' to a double bit frequency signal and a logic '0' to as single bit frequency signal. In one exemplary embodiment, logic '1' may be converted to two (2) logic level transitions between the data clock signal (double frequency) and logic '0' may be converted to one (1) transition (single frequency). The word data in the DSP **1020** may be synchronized with the FTC **1035's** counter through Frame Counter Starter (FCS) (not shown). The FTC **1035** may also have logic circuit for decoding a specific pattern of 1's and zeros (for example, 0100 1110 1010 0011) that indicate the beginning of a forward telemetry frame. As soon as the FTC **1035** receives a predefined word, FTC **1035's** line counter begins to count down and is ready to accept the forward telemetry data. In one exemplary embodiment, the FTC **1035** may have an FTC reset functionality that causes the FTC **1035** to reset upon VPU **20's** start up.

The Back Telemetry Controller (BTC) **1040** retrieves the biphase marked data from the RF receiver **1065,** decodes it, and generates the **BFSR** and bit clock **(BCLKR)** for the DSP **1020's** McBSP2 interface. The **BCLKR** may be generated based on a decoded biphase marked data. In one exemplary embodiment, the biphase marked data may be received from the retinal stimulation system **1** and may be converted similarly to FTC **1035.** The Back Telemetry Controller (BTC) **1040** may also have a header detector (not shown) that monitors decoded data for predefined word header, for example, "11111111111111110." Upon detection of the predefined word header, a back telemetry word frame **BFSR** signal may be pulsed. If the **BFSR** signal occurs greater than 32 bits apart, an error bit may be set. The **BFSR** and **BCLKR** signal DSP **1020's** McBSP2 port to receive data on the **BDR2** input. The date rate on the **BDR2** input may be 3.84±0.2 Kbps. Error bits may be set of the rate of modulation state change too fast or too slow.

The Input/Output Ports **1045** provide expanded IO functions to access the CPLD on-chip and off-chip devices. An InputPort1 (see Table 1) of the Input/Output Ports **1045** may have an address IOS 4000h and may provide off chip input access. An InputPort2 (see Table 2) of the Input/Output Ports **1045** may have an address IOS 4001 h and may provide off chip input access. A VideoDataInput (see Table 3) of the Input/Output Ports **1045** may have an address IOS 4002h and may provide on chip input access. A CPLD_Version (see Table 4) of the Input/Output Ports **1045** may have an address IOS 4003h and may provide on chip input access. A VideoLineCount (see Table 5) of the Input/Output Ports **1045** may have an address IOS 4004h and may provide on chip input access. An OutputPort1 (see Table 6) of the Input/Output Ports **1045** may have an address IOS 4008h and may provide on chip and off chip output access. The initial value of the OutputPort1 may be 0000h at system reset. An OutputPort2 (see Table 7) of the Input/Output Ports **1045** may have an address IOS 400Ah and may provide on chip output access. The initial value OutputPort2 may be 0001h at system reset. An ADconvst (see Table 8) of the Input/Output Ports **1045** may have an address IOS 6000h and may provide off chip output access. An ADCeN (see Table 9) of the Input/Output Ports **1045** may have an address IOS 8000H ~8003H and may provide off-chip input access. An OutputPort3 (see Table 10) of the Input/Output Ports **1045** may have an address IOS 400Bh and may provide off-chip output access. The initial value of the

OutputPort3 may be 0000h at system reset. A KeypadInput (see Table 11) of the Input/Output Ports **1045** may have an address IOS A000h and may provide off chip input access.

**Table 1. InputPort1 Definition**

| **Bit** | **Function** |
|---|---|
| 0 | Reserved |
| 1 | Frame Sync Slow Detect 1. 1 = Slow Frame Sync Detected (greater than 32 bits apart), 0 = Slow Frame Sync not detected. Write a zero to this bit or system reset to clear it. |
| 2 | Slow Modulation rate detect (greater than 348 usec per state). 1 = Slow modulation rate detected, 0 = Slow modulation rate not detected. Write a zero to bit 2 or bit 3 at this address or system reset to clear this bit. |
| 3 | Fast Modulation rate detect (between 43.5 and 87 usec per state). 1 = Fast modulation rate detected, 0 = Fast modulation rate not detected. Write a zero to bit 2 or bit 3 at this address or system reset to clear this bit. |
| 4 | Watchdog status. 1 = Watchdog timer has not expired. 0 = Watchdog timer has expired. This bit is set to the 1 state when the watchdog timer is subsequently reset. |
| 5 | 0 |
| 6 | 0 |
| 7 | 0 |

**Table 2. InputPort2 Definition**

| **Bit** | **Function** |
|---|---|
| 0 | CA detection. 1 = CA connection is detected, 0 = CA connection is not detected. |
| 1 | Low Battery Detection. 1 = Normal battery voltage, 0 = Low Battery is detected. |
| 2 | Reserved |
| 3 | Connect Verify. 1 = Camera/RF connector not connected, 0 = Camera/RF connector connected. |
| 4 | Utility Key 8. 0 = Key not depressed, 1 = Key depressed |
| 5 | Program/Run Switch Position. 1 = Switch in Program position, 0 = Switch in Run position. |
| 6 | Reserved |
| 7 | Reserved |

**Table 3. VideoDataInput Definition**

| **Bit** | **Function** |
|---|---|
| 0-7 | Video data input port |

**Table 4. CPLD_Version Definition**

| **Bit** | **Function** |
|---|---|
| 0~7 | CPLD Version number, 8 bits |

**Table 5. VideoLine Count Definition**

| **Bit** | **Function** |
|---|---|
| 0-7 | NTSC line counter. An 8-bit counter to count the NTSC line |

**Table 6. OutputPort1 Definition**

| **Bit** | **Function** |
|---|---|
| 0-4 | RF power control. 00000 = 4.75 +/- 0.3V, 11111 1 = 10.5 +/-0.3V |
| 5 | System Running LED Indicator. 1 = turn the System Running LED on, 0 = turn the System Running LED off. |
| 6 | RF Link LED Indicator. 1 = turn the RF Link LED on, 0 = turn the RF Link off. |
| 7 | Camera Disconnect LED Indicator. 1 = turn the LED on, 0 = turn the LED off |

**Table 7. OutputPort2 Definition**

| **Bit** | **Function** |
|---|---|
| 0 | freeVideo, the VPDI coupling signal. 1= VPDI is disabled, 0 = VPDI is enabled |
| 1-2 | Reserved |
| 3 | Shutdown, normally 0, set to 1 for at least 1 msec and then back to 0 to force a system shutdown |
| 4 | Reserved |
| 5-6 | Forward Telemetry BFSX signal start signal, 11 = command the BFSX to generate the word frame signal, 00 = no BFSX word frame signal. May be changed from 00 to 11 within one system bus cycle |
| 7 | Carrier enable. 1= enable the carrier clock to the RF circuit and the BCLK to McBSP2, 0 = disable carrier clock and BCLK which are forced to output a low signal state |

**Table 8. ADconvst Definition**

| **Bit** | **Function** |
|---|---|
| 0∼7 | AD converter sampling start trigger signal. Access of this port does not cause any data transfer between DSP, CPLD and AD converter. A low-active 50-300 nsec pulse from a dummy read access is used to trigger the AD converter to sample the signal |

**Table 9. ADCeN Definition**

| **Address** | **Bit** | **Function** |
|---|---|---|
| 8000H | 0-7 | Read present A/D data and select Channel 1 (the Vrf current) for the subsequent A/D sample. Each count for channel 1 data may represent 2.35mA; the range may be 0 to 602mA |
| 8001 H | 0-7 | Read present A/D data and select Channel 2 (the Vrf voltage) for the subsequent A/D sample. Each count for channel 2 data may represent 43.32 mV; the range may be 0 to 11.05V |
| 8002H | 0-7 | Reserved |
| 8003H | 0~7 | Reserved |

**Table 10. OutputPort3 Definition**

| **Bit** | **Function** |
|---|---|
| 0 | A/D converter power up/down, 0= power down, 1= power up |
| 1 | Vrf control, 0= Vrf powered off, 1 = Vrf powered on |
| 2 | Watchdog counter reset signal. The watchdog counter is reset when the state of this bit is changed |
| 3~5 | Reserved |
| 6 | Buzzer control, 1 = turn the buzzer on , 0 = turn the buzzer off |
| 7 | Reserved |

**Table 11. KeypadInput Definition**

| Bit | **Function** |
|---|---|
| 0 | Utility Key 0. 1 = Key not depressed, 0 = Key depressed |
| 1 | Utility Key 1. 1 = Key not depressed, 0 = Key depressed |
| 2 | Utility Key 2. 1 = Key not depressed, 0 = Key depressed |
| 3 | Utility Key 3. 1 = Key not depressed, 0 = Key depressed |
| 4 | Utility Key 4. 1 = Key not depressed, 0 = Key depressed |
| 5 | Utility Key 5. 1 = Key not depressed, 0 = Key depressed |
| 6 | Utility Key 6. 1 = Key not depressed, 0 = Key depressed |
| 7 | Utility Key 7. 1 = Key not depressed, 0 = Key depressed |

In one exemplary embodiment, the buzzer (Table 10) may be from Soberton Inc. part number ST-03BL audio buzzer that operates at 2.3 KHz. The buzzer may provide a sound pressure level of 68 - 82 dB at 5 cm distance without an enclosure.

The VPU **20** shown in **Figures 11-12** and **13-1-13-4** may have attachable/detachable ports **815-816** shown in **Figures 11-12** to connect several peripheral units. The connectors are classified as functional connectors, e.g. port **815** and development connectors. The functional connectors link the functional peripheral devices, for example, Glasses **5** of **Figure 6** to the VPU **20** to furnish a production level function for the system. The development connectors may be used when the system is under development phase.

The Power Connector 821 shown in **Figures 11F****-11G** (connector under the battery **25** in **Figure 11A****)** is a functional connector that connects the battery **25** to the VPU **20.**

Port **815** is a functional Camera/RF Connector. Port **815** may contain 12 pins (identified in Table 12 below) to provide connection for the camera **12** and external coil **14** on the Glasses **5.** The port **815** may be protected such that port **815** will withstand an indefinite short to the camera **12's** power. The power connector and the port **815** may be mechanically keyed to prevent improper installation.

**Table 12. Camera/RF Connector Pin Definition**

| **Pin** | **Name** | **Type** | **Function** |
|---|---|---|---|
| 1 | Vrf | Power | RF voltage source |
| 2 | RF_GND | Power | Ground return for RF voltage source |
| 3 | BK_TEL | Input | Back Telemetry Data from RF Board |
| 4 | Carrier | Output | Carrier signal to RF Board |
| 5 | Data | Output | Forward Telemetry data to RF Board |
| 6 | Connect Verify | Input | Status pin for verification of Camera/RF connection |
| 7 | DGND | Power | Ground return for signals on pin 3 through 6 and pin 8 |
| 8 | Equip Sync | Output | |
| 9 | Camera Power | Power | Power for the camera. Equal to the battery voltage, and is switched off when the system power supplies are shut down |
| 10 | AGND | Power | Ground return for camera power |
| 11 | Video In | Input | Video input from the camera |
| 12 | AGND | Power | Ground return for the camera video |

The Parallel Flash Memory (PFM) **1050** may be used to store executable code and the Serial Flash Memory (SFM) **1055** may provide Serial Port Interface **SPI** for data storage. The PFM **1050** may be implemented using, for example, Intel TE28F160B3TD70. The PFM **1050** is connected to the address and data bus. The PFM may contain 512K words to map to an executable code space (PS) of the DSP **1020.** A user-selectable switch ( not shown) may be provided to enable or disable writing to the PFM **1050.** The SFM **1055** may be implemented using, for example, STMicroelectronics M25P80. The SFM **1055** may contain 16 independently erasable sectors, with each sector containing, for example, 64K bytes.

The Real Time Clock **1060** may be battery backed up real time clock that is connected to the I2C bus. The Real Time Clock **1060** may be implemented using, for example, Xicor X1226 that uses a 32.768 KHz crystal and draws 600 nA from a 48 mAH primary lithium cell to keep the clock running when the VPU **20** is powered off. The Real Time Clock **1060** may contain 512 bytes of flash EEPROM which may be programmed or read via the I2C bus.

The VIMC **1071** may be used to monitor the integrity status of the retinal stimulation system **1** by sampling and monitoring RF transmitter **1070's** current and voltage. The RF transmitter **1070's** current may be converted to voltage through a current sensitive resistor (not shown) of 100mohm +/- 2%. The voltage drop across the sensitive resister is amplified by a current shunt monitor to 0 ~ 2.0V of dynamic range. The voltage is buffered for AD converting. Vrf may also be sent through a resistor divider for AD converting. The AD converter may have resolution of 8 bits with a signal input range of 0~2V. The conversion accuracy of the AD converter for measuring RF transmitter current and Vrf may be, for example, ±3.6%.

Four external interrupt signals **INT0** through **INT3** may be input to the DSP **1020.** The INTO may have the highest priority and **INT3** may have the lowest. The **INTO** may be connected to the vertical sync output of the Video Preprocessor **1075** to signal the start of a new video frame. The INT1 may be connected to the Keypad interface of the IOP **1045** to signal when a key is depressed or released. The **INT2** may be optional. The **INT3** may be connected to signal ITRDY of the VPDI **1025.**

Port **816** of **Figures 11A-E** is an Omnetics Connector that is used to connect the VPU **20** to the laptop **10** though the Communication Adapter (CA) **40** of **Figure 9****.** The Omnetics Connector may have 10 pins (identified in Table 13 below).

**Table 13. Omnetics Connector Pin Definition**

| **Pin** | **Name** | **Type** | **Function** |
|---|---|---|---|
| 1 | Transmit Data | Output | Transmit data to CA **40** |
| 2 | Vcc | Input | +3.3V for power to VPU side of CA opto-isolators |
| 3 | Ground | Power | Ground connection to CA |
| 4 | DataCLK | Output | Data clock from CA |
| 5 | Receive Data | Output | Received data from CA |
| 6 | N/C | Input | No connection |
| 7 | FSR | Input | Frame Sync for receive data from CA |
| 8 | Connect Verify | | Line to verify connection of the CA (low = connected, float = not connected) |
| 9 | N/C | | No connection |
| 10 | N/C | | No connection |

Keypad Connector is a 14-conducter flat connector for in-system keypad and LED connection.

The following are examples of Development Connectors according to one exemplary embodiment of the present application. A Programmable Logic Download Connector is a development connector for downloading the .jed file from Xilinx WebPack to on-board CPLD. The Programmable Logic Download Connector is compliant to JTAG, which is the commonly used acronym for the Boundary Scan Test (BST) feature defined for integrated circuits by IEEE Standard 1149.1. This standard defines input/output pins, logic control functions, and commands that facilitate both board and device level testing without the use of specialized test equipment. A DSP JTAG Connector is a development connector for linking the digital board to the DSP 1020 development system - Code Composer Studio. The DSP JTAG Connector is compliant to IEEE standard 1149.1. A Equipment Sync is a signal that is available through the Camera/RF connector port **815.** The Equipment Sync provides a pulse (width 1 ms, positive going and 3.3V amplitude) to indicate that the first 16-bit word of a 1024 bit packet is being output by the forward telemetry logic.

The power button **805** of **Figure 12** may be used for turning the VPU **20** on/off. In one exemplary embodiment, the power button **805** may be depressed for a predetermined amount of time (for example 1.6 ± 0.2 seconds) before the VPU **20** actually powers up. In another exemplary embodiment, the power button **805** may be depressed for another predetermined amount of time (for example 1.6 ± 0.2 seconds) before the VPU **20** actually powers down.

In one exemplary embodiment, a CMOS sensor **1073** that provides digital output may be used instead of the camera **12** as shown in **Figures 14-1** to **14-4.** The CMOS sensor **1073** may have within it, or be connected to a high-speed digital serial transmitter circuit such as one using the Low Voltage Differential Signal (LVDS) protocol. This serial data may be passed through the multi-conductor cable to an LVDS receiver **1072,** which would be used in place of the video decoder circuit **1075.** The receiver **1072** may perform serial-to-parallel conversion of the video data and thus provide the digital video stream to the VDPI circuit **1025.** This embodiment may allow for elimination of the video decoder and may allow better control of the CMOS sensor **1073** via the programmable registers available within CMOS sensor **1073.**

Due to the nature of the video data being processed, it may be advantageous to use a digital signal processor (DSP) **1020** for micro-controller. DSPs **1020** are more adept at applying digital filtering algorithms to the incoming video frames. For functions such as real-time control of the voltage to the RF circuit **1501** or handling the user interface, however, it is easier to program a general-purpose micro-controller (such as the ARM). It may be possible to use a so-called "dual core" device containing both an ARM (or other general purpose micro-controller) and a DSP. Each processor may be assigned to handle the functions they best serve. To save space, it may be possible to select a micro-controller that has the VDPI **1025** circuit built into it.

As used in this specification and the appended claims, the singular forms "a," "an," and "the" include plural referents unless the content clearly dictates otherwise. The term "plurality" includes two or more referents unless the content clearly dictates otherwise. Unless defined otherwise, all technical and scientific terms used herein have the same meaning as commonly understood by one of ordinary skill in the art to which the disclosure pertains.

Accordingly, what has been shown is an improved visual prosthesis, improved method of stimulating neural tissue and an improved method for controlling a visual prosthesis. While the invention has been described by means of specific embodiments and applications thereof, it is understood that numerous modifications and variations could be made thereto by those skilled in the art without departing from the scope of the invention. It is therefore to be understood that. within the scope of the claims, the invention may be practiced otherwise than as specifically described herein.

## Claims

1. A visual prosthesis apparatus comprising:
a video capture device (5) for capturing a video image;
a video processing unit (20) associated with the video capture device, the video processing unit configured to convert the video image to stimulation patterns, the video processing unit (20) including a power button (805) having a different shape from any other buttons on the video processor and an indicator light (825); and
a retinal stimulation system (1) configured to stimulate neural tissue in a subject's eye based on the stimulation patterns,
wherein the video processing unit (20) is configured to be powered on after a first time interval upon activation of the power button and to provide a first audio indication after said first time interval,
wherein the video processing unit (20) is configured to be powered off after a second time interval upon activation of the power button and to provide a second audio indication different from the first audio indication after said second time interval; and
wherein the indicator light (825) provides different signals to indicate: the video processing unit (20) is going through system start-up diagnostic testing; the video processing unit (20) is operating normally; a problem detected during the start-up diagnostic testing by the video processing unit (20); and the video processing unit (20) detects a problem with the retinal stimulation system; and
wherein, the video processing unit (20) shuts off power to the retinal stimulation system if it detects a problem with the retinal stimulation system.

2. The visual prosthesis apparatus of Claim 1, wherein the first time interval is about 1.6 seconds.

3. The visual prosthesis apparatus of Claim 1 or 2, wherein the second time interval is about 1.6 seconds.

4. The visual prosthesis apparatus of any one of Claims 1 to 3, wherein the video processing unit is disposed on a visor (5).

5. The visual prosthesis apparatus of any one of Claims 1 to 3, wherein the video processing unit (20) further comprises:
a video processor (1075) for converting the video image to a digital video stream;
a memory (1050) for storing the digital video stream;
a video preprocessor data interface for forming stimulation patterns based on the stored digital video stream.

6. The visual prosthesis apparatus of Claim 5, wherein the video processing unit further comprises a power supply distribution and monitoring circuit (1005) for generating appropriate voltages and adjusting power level of the stimulation patterns transmitted to the retinal stimulation system.

7. The visual prosthesis apparatus of Claim 6, wherein the power supply distribution and monitoring circuit (1005) is configured to turn off the video processing unit (20) if the power from a battery providing energy to the video processing unit drops below a predetermined level.

8. The visual prosthesis apparatus of any one of Claims 5 to 7, wherein the video processing unit (20) further comprises a reset circuit (1010) for resetting the video processing unit.

9. The visual prosthesis apparatus of Claim 8, wherein the reset circuit (1010) resets the video processing unit (20) based on manual push-button reset, watchdog timer expiration or firmware based shutdown.

10. The visual prosthesis apparatus of any one of Claims 5 to 9, wherein the video processing unit (20) further comprises a digital signal processor (1020) for communicating with the video processor, the memory and the video preprocessor data interface.

11. The visual prosthesis apparatus of Claim 10, wherein the video processing unit further comprises a system main clock (SMC) for the digital signal processor.

12. The visual prosthesis apparatus of any one of Claims 4 to 11, wherein the video processor comprises:
a forward telemetry controller (1035) forming a forward telemetry frame for the retinal stimulation system; and
a back telemetry controller (1040) decoding backward telemetry frame from the retinal stimulation system.

13. The visual prosthesis apparatus of any one of Claims 4 to 12, wherein the video processor comprises:
a voltage and current monitoring circuit (1005) configured to monitor the integrity status of the retinal stimulation system by sampling and monitoring current and voltage of an RF transmitter.

14. The visual prosthesis apparatus of any one of Claims 4 to 13, wherein the video capturing device is a CMOS sensor (1073).

15. The visual prosthesis apparatus of any one of Claims 4 to 14, wherein the video processor comprises:
a digital signal processor (1020) configured to apply digital filtering algorithms to the video image; and
a general-purpose micro-controller configured to provide real-time control of voltage to an RF circuit (1501) or configured to control a user interface.

## Patentansprüche

1. Visuelle Prothesenvorrichtung, umfassend:
eine Videoaufnahmevorrichtung (5) zum Aufnehmen eines Videobildes;
eine Videoverarbeitungseinheit (20), welche der Videoaufnahmevorrichtung zugeordnet ist, wobei die Videoverarbeitungseinheit konfiguriert ist, um das Videobild in Stimulationsmuster umzuwandeln, wobei die Videoverarbeitungseinheit (20) einen Einschaltknopf (805), welcher eine Form aufweist, die sich von jeglichen anderen Knöpfen auf dem Videoprozessor unterscheidet, sowie ein Hinweislicht (825) aufweist; und
ein Retinastimulationssystem (1), welches konfiguriert ist, um auf Basis der Stimulationsmuster Nervengewebe im Auge eines Individuums zu stimulieren,
worin die Videoverarbeitungseinheit (20) konfiguriert ist, um bei Aktivierung des Einschaltknopfes nach einem ersten Zeitintervall eingeschaltet zu werden und einen ersten akustischen Hinweis nach dem ersten Zeitintervall bereitzustellen,
worin die Videoverarbeitungseinheit (20) konfiguriert ist, um bei Aktivierung des Einschaltknopfes nach einem zweiten Zeitintervall ausgeschaltet zu werden und einen zweiten akustischen Hinweis, welcher sich von dem ersten akustischen Hinweis unterscheidet, nach dem zweiten Zeitintervall bereitzustellen; und
worin das Hinweislicht (825) unterschiedliche Signale bereitstellt, um anzuzeigen: dass die Videoverarbeitungseinheit (20) einen Systemstartdiagnosetest durchläuft; die Videoverarbeitungseinheit (20) ordnungsgemäß funktioniert; während des Startdiagnosetests ein Problem von der Videoverarbeitungseinheit (20) detektiert wurde; und die Videoverarbeitungseinheit (20) ein Problem mit dem Retinastimulationssystem detektiert; und
worin die Videoverarbeitungseinheit (20) den Strom an das Retinastimulationssystem abschaltet, wenn sie ein Problem mit dem Retinastimulationssystem detektiert.

2. Visuelle Prothesenvorrichtung nach Anspruch 1, worin das erste Zeitintervall etwa 1,6 Sekunden ist.

3. Visuelle Prothesenvorrichtung nach Anspruch 1 oder 2, worin das zweite Zeitintervall etwa 1,6 Sekunden ist.

4. Visuelle Prothesenvorrichtung nach einem der Ansprüche 1 bis 3, worin die Videoverarbeitungseinheit auf einem Visier (5) angeordnet ist.

5. Visuelle Prothesenvorrichtung nach einem der Ansprüche 1 bis 3, worin die Videoverarbeitungseinheit (20) ferner Folgendes umfasst:
einen Videoprozessor (1075) zum Umwandeln des Videobildes in einen digitalen Videodatenstrom;
einen Arbeitsspeicher (1050) zum Speichern des digitalen Videodatenstroms;
eine Videoprozessor-Datenschnittstelle zum Bilden von Stimulationsmustern auf Basis des gespeicherten digitalen Videodatenstroms.

6. Visuelle Prothesenvorrichtung nach Anspruch 5, worin die Videoverarbeitungseinheit ferner eine Leistungsversorgungsverteilungs- und -überwachungsschaltung (1005) umfasst, um geeignete Spannungen zu erzeugen und den Leistungspegel der Stimulationsmuster einzustellen, die an das Retinastimulationssystem gesendet werden.

7. Visuelle Prothesenvorrichtung nach Anspruch 6, worin die Leistungsversorgungsverteilungs- und -überwachungsschaltung (1005) konfiguriert ist, um die Videoverarbeitungseinheit (20) auszuschalten, wenn die Leistung von einer Batterie, welche der Videoverarbeitungseinheit Energie bereitstellt, unter einen vordefinierten Pegel fällt.

8. Visuelle Prothesenvorrichtung nach einem der Ansprüche 5 bis 7, worin die Videoverarbeitungseinheit (20) ferner eine Rücksetzschaltung (1010) umfasst, um die Videoverarbeitungseinheit zurückzusetzen.

9. Visuelle Prothesenvorrichtung nach Anspruch 8, worin die Rücksetzschaltung (1010) die Videoverarbeitungseinheit (20) auf Basis einer manuellen Knopfbetätigungsrücksetzung, des Ablaufs eines Watchdog-Timers oder eines firmwarebasierten Herunterfahrens zurücksetzt.

10. Visuelle Prothesenvorrichtung nach einem der Ansprüche 5 bis 9, worin die Videoverarbeitungseinheit (20) ferner einen digitalen Signalprozessor (1020) zum Kommunizieren mit dem Videoprozessor, dem Arbeitsspeicher und der Video-Vorprozessor-Datenschnittstelle umfasst.

11. Visuelle Prothesenvorrichtung nach Anspruch 10, worin die Videoverarbeitungseinheit ferner einen System-Haupttaktgeber (SMC) für den digitalen Signalprozessor umfasst.

12. Visuelle Prothesenvorrichtung nach einem der Ansprüche 4 bis 11, worin der Videoprozessor Folgendes umfasst:
eine Vorwärtstelemetriesteuerung (1035), welche einen Vorwärtstelemetrierahmen für das Retinastimulationssystem ausbildet, und
eine Rückwärtstelemetriesteuerung (1040), welche den Rückwärtstelemetrierahmen vom Retinastimulationssystem dekodiert.

13. Visuelle Prothesenvorrichtung nach einem der Ansprüche 4 bis 12, worin der Videoprozessor Folgendes umfasst:
eine Spannungs- und Stromüberwachungsschaltung (1005), welche konfiguriert ist, um den Integritätszustand des Retinastimulationssystems zu überwachen, indem sie den Strom und die Spannung eines HF-Senders abtastet und überwacht.

14. Visuelle Prothesenvorrichtung nach einem der Ansprüche 4 bis 13, worin die Videoaufnahmevorrichtung ein CMOS-Sensor (1073) ist.

15. Visuelle Prothesenvorrichtung nach einem der Ansprüche 4 bis 14, worin der Videoprozessor Folgendes umfasst:
einen digitalen Signalprozessor (1020), welcher konfiguriert ist, um digitale Filteralgorithmen auf das Videobild anzuwenden; und
eine Mehrzweck-Mikrosteuereinheit, die konfiguriert ist, um eine Echtzeitsteuerung der Spannung für eine HF-Schaltung (1501) bereitzustellen, oder konfiguriert ist, um eine Benutzerschnittstelle zu steuern.

## Revendications

1. Prothèse visuelle qui comprend :
un dispositif de capture vidéo (5) destiné à capturer une image vidéo ;
une unité de traitement vidéo (20) associée au dispositif de capture vidéo, l'unité de traitement vidéo étant configurée pour convertir l'image vidéo en modèles de stimulation, l'unité de traitement vidéo (20) comprenant un bouton d'alimentation (805) qui possède une forme différente des autres boutons du processeur vidéo et un témoin lumineux (825) ; et
un système de stimulation rétinienne (1) configuré pour stimuler le tissu nerveux dans l'oeil d'un sujet, sur la base des modèles de stimulation,
dans laquelle l'unité de traitement vidéo (20) est configurée pour être mise sous tension après un premier intervalle de temps lors de l'activation du bouton d'alimentation, et pour fournir une première indication audio après ledit premier intervalle de temps,
dans laquelle l'unité de traitement vidéo (20) est configurée pour être éteinte après un second intervalle de temps lors de l'activation du bouton d'alimentation, et pour fournir une seconde indication audio différente de la première indication audio après ledit second intervalle de temps ; et
dans laquelle le témoin lumineux (825) fournit différents signaux afin d'indiquer : que l'unité de traitement vidéo (20) effectue un test de diagnostic de démarrage du système ; que l'unité de traitement vidéo (20) fonctionne normalement ; qu'un problème a été détecté pendant le test de diagnostic de démarrage par l'unité de traitement vidéo (20) ; et que l'unité de traitement vidéo (20) détecte un problème avec le système de stimulation rétinienne ; et
dans laquelle l'unité de traitement vidéo (20) éteint le système de stimulation rétinienne si elle détecte un problème avec le système de stimulation rétinienne.

2. Prothèse visuelle selon la revendication 1, dans laquelle le premier intervalle de temps est d'environ 1,6 seconde.

3. Prothèse visuelle selon la revendication 1 ou 2, dans laquelle le second intervalle de temps est d'environ 1,6 seconde.

4. Prothèse visuelle selon l'une quelconque des revendications 1 à 3, dans laquelle l'unité de traitement vidéo est disposée sur une visière (5).

5. Prothèse visuelle selon l'une quelconque des revendications 1 à 3, dans laquelle l'unité de traitement vidéo (20) comprend en outre :
un processeur vidéo (1075) destiné à convertir l'image vidéo en un flux vidéo numérique ;
une mémoire (1050) destinée à stocker le flux vidéo numérique ;
une interface de données de préprocesseur vidéo destinée à former des modèles de stimulation sur la base du flux vidéo numérique stocké.

6. Prothèse visuelle selon la revendication 5, dans laquelle l'unité de traitement vidéo comprend en outre un circuit de distribution et de surveillance d'alimentation en énergie (1005) destiné à générer des tensions appropriées et à ajuster le niveau de puissance des modèles de stimulation transmis au système de stimulation rétinienne.

7. Prothèse visuelle selon la revendication 6, dans laquelle le circuit de distribution et de surveillance d'alimentation en énergie (1005) est configuré pour éteindre l'unité de traitement vidéo (20) si l'énergie qui provient d'une batterie qui fournit de l'énergie à l'unité de traitement vidéo chute en-dessous d'un niveau prédéterminé.

8. Prothèse visuelle selon l'une quelconque des revendications 5 à 7, dans laquelle l'unité de traitement vidéo (20) comprend en outre un circuit de réinitialisation (1010) destiné à réinitialiser l'unité de traitement vidéo.

9. Prothèse visuelle selon la revendication 8, dans laquelle le circuit de réinitialisation (1010) réinitialise l'unité de traitement vidéo (20) sur la base d'une réinitialisation manuelle d'un bouton-poussoir, de l'expiration d'un temporisateur, ou d'un arrêt provoqué par un firmware.

10. Prothèse visuelle selon l'une quelconque des revendications 5 à 9, dans laquelle l'unité de traitement vidéo (20) comprend en outre un processeur de signal numérique (1020) destiné à communiquer avec le processeur vidéo, la mémoire et l'interface de données de préprocesseur vidéo.

11. Prothèse visuelle selon la revendication 10, dans laquelle l'unité de traitement vidéo comprend en outre une horloge système principale (SMC) pour le processeur de signal numérique.

12. Prothèse visuelle selon l'une quelconque des revendications 4 à 11, dans laquelle le processeur vidéo comprend :
un contrôleur de télémétrie avancé (1035) qui forme une trame de télémétrie avancée pour le système de stimulation rétinienne ; et
un contrôleur de télémétrie arrière (1040) qui décode la trame de télémétrie avancée qui provient du système de stimulation rétinienne.

13. Prothèse visuelle selon l'une quelconque des revendications 4 à 12, dans laquelle le processeur vidéo comprend :
un circuit de surveillance de tension et de courant (1005) configuré pour surveiller le statut d'intégrité du système de stimulation rétinienne en échantillonnant et en surveillant le courant et la tension d'un transmetteur RF.

14. Prothèse visuelle selon l'une quelconque des revendications 4 à 13, dans laquelle le dispositif de capture vidéo est un capteur CMOS (1073).

15. Prothèse visuelle selon l'une quelconque des revendications 4 à 14, dans laquelle le processeur vidéo comprend :
un processeur de signal numérique (1020) configuré pour appliquer des algorithmes de filtrage numérique à l'image vidéo ; et
un microcontrôleur à usage général configuré pour assurer un contrôle en temps réel de la tension d'un circuit RF (1501), ou configuré pour contrôler une interface utilisateur.
